# EUROPEAN PATENT SPECIFICATION

(11) **EP 1 534 230 B1**
(45) Date of publication and mention of the grant of the patent: **09.12.2009**
(21) Application number: 03763769.1
(22) Date of filing: 08.07.2003
(51) Int. Cl.: A61K 8/39, A61K 8/37, A61K 8/362, A61K 8/34, A61K 8/92, A61Q 19/00, A61Q 19/10

(54) **DRY PRODUCTS COMPRISING AN APPLICATOR AND A WAX PHASE**
TROCKENPRODUKTE MIT EINEM APPLIKATOR UND EINER WACHSPHASE
PRODUITS SECS CONTENANT UNE CIRE ET POURVUS D'UN APPLICATEUR

(30) Priority: 12.07.2002 EP 02077859; 13.01.2003 EP 03075132
(43) Date of publication of application: 01.06.2005
(73) Proprietor: Johnson & Johnson GmbH, 40474 Düsseldorf (DE)
(72) Inventor: HAUSER, Matthias, 53757 Sankt Augustin (DE); ANSMANN, Achim, 40699 Erkrath (DE); ISSBERNER, Ulrich, 19002 Ambler, PA (US); JACKWERTH, Bettina, 40764 Langenfeld (DE); LEONARD, Mark, Bexley Kent DA5 1AZ (GB)
(74) Representative: Metten, Karl-Heinz
(86) International application number: PCT/EP2003/007398
(87) International publication number: WO 2004/006880

(56) References cited:
- DE-A- 19 911 041
- GB-A- 2 183 461
- US-A- 4 127 515
- US-A- 5 177 831
- US-A- 5 605 749
- US-B1- 6 258 965

## Description

### Field of the Invention

This invention concerns products for cleansing and other applications, which products comprise an applicator such as a puff (pouf), pad, sponge, cotton ball, swab, brush, glove, mitt or bar, to which a wax phase has been applied. The invention further concerns the manufacture and use of such products.

### Background of the Invention

A plurality of applicators for delivering commodities to a surface have been developed, such applicators being of varied nature, in as well presentation as material selection, e.g. applicators that are resilient or non-resilient, or that are re-usable or disposable. Such applicators have been used to apply to surface ingredients in the form of creams, pastes, gels, liquids, powders and the like. In particular such applicators have been used to apply topical preparations to the skin such as cosmetic, dermatological and the like products. Applicators have been used with a separate product supply or have been impregnated or coated with a measured quantity of product.

One particular type of applicators are wipes, which have become an important product category that has found a wide variety of applications for adults and babies. Examples include face or body cleansing wipes, wipes for skin treatment, and skin conditioning wipes. So-called wet wipes have become successful as products particularly suited for these applications.

Developments in the wipe area were focused on the wipe itself, as well as on the wipe material and on the lotions applied thereto. Lotions have been developed which offered skincare benefits in addition to the basic cleansing properties of the wipe.

However, these approaches still leave room for improvement. Firstly, only a small portion of the lotion is released from the wipes during use. Thus a large quantity of the relatively expensive lotion is not delivered to the skin providing no benefit to the consumer and is wasted when the product is discarded after use. This also prevents the use of expensive but more effective ingredients. Secondly, from a formulation point there is an apparent contradiction in the optimization of cleansing performance and skincare benefits in one single lotion, since ingredients which are effective in cleansing usually are not compatible with efficient skin care agents.

Another important factor in cleansing is the fact that a number of soils are water-compatible and therefore more easily removed by water-based formulations, whereas others are lipid-compatible and therefore adequately removed by lipid or oil based formulations. A complete and effective removal of soils therefore requires the presence in or on a wipe of as well water as oil-based components.

This is in particular required in products for personal cleansing and in particular in products used for babies and infants. Inadequate cleaning not only results in personal discomfort but also gives rise to diaper rash and other infection related phenomena. It has been shown that the most effective way of preventing diaper rash is to cleanse the skin thoroughly and to remove the microorganisms that have been identified as causative. The source of these microorganisms is often the fecal deposits that can remain on a baby's skin while wearing the diaper. Because fecal deposits consist of both water-soluble and oil-soluble matter, however, complete removal of fecal deposits from the diaper area requires both water-based and oil-based cleansing agents.

US-4,987,632 discloses a substantially dry-to-the-touch wiping article for use in cleaning soiled surfaces wherein moisture barriers cover the surface of the sheet. WO 99/13861 and US-6,153,208 disclose substantially dry personal cleansing articles wherein the substrate comprises multiple layers. US-6,280,757 concerns cleansing articles that are dry comprising a substrate having apertures of certain size and frequency.

### Summary of the Invention

This invention relates to products that comprise an applicator, other than a porous or absorbent sheet, according to the features of claim 1 for transferring ingredients to surfaces and in particular to the skin.

In a particularly preferred embodiment, the wax phase is a wax composition which also comprises an active ingredient.

Or, this invention relates to products that comprise an applicator, other than a porous or absorbent sheet, whereto a wax phase has been applied and which products are dry or essentially dry. Dry refers to the situation where the water content is low, e.g. lower than 1 % and essentially dry means that the product contains limited amounts of water, e.g. less than 10 % , preferably less than 8 %, more preferably less than 5 %, still more preferably less than 2 %. All percentages in this paragraph are relative to the total weight of the product.

Preferably, the wax phase is present at the surface or at the surface portion of one or several sides of the applicator.

The wax phase preferably has a low water content, in particular lower than 10%. The wax phase preferably contains one or more active ingredients.

In particular said applicator is any three-dimensional substrate capable of transferring ingredients to a surface, in particular the user's skin. Examples of such substrates are puffs, pads, sponges, bars, brushes, cotton balls, gloves, mitts, or cotton tipped swabs. The applicators may be made of a variety of materials which are structured such that they are capable of holding and/or absorbing a wax phase. The materials of which the applicators are made therefore maybe porous or absorbent in nature. The materials in particular are polymeric and may be both from natural and non-natural origin.

In a further aspect there is provided a method of manufacturing a product as described herein, said method comprising applying to the applicator a wax phase.

In still another aspect the invention provides the use of a product as described herein as a combined cleanser and applicator of active substances.

### Detailed Description of the Invention

The applicator in the products according to this invention can be resilient or non-resilient. The applicator can be used as such or can have a suitable handle. It can take any tridimensional form that is suited for application to flat surfaces including the skin. The applicators can be of different size and take a variety of forms, e.g. flat or not, geometrically shaped or not, round which includes cylindrical, ellipsoidal, spherical and the like shapes, or angular shaped such as square or rectangular, which includes cubic or bar shapes, also with rounded edges or combinations of these shapes. One or more of the outer sides of the applicator may be made of different materials having different properties. For example one side may be soft while another side is rougher. The latter side can be abrasive, it can be used for rubbing or scouring. The applicators can be hard, soft, semi-soft, resilient or not, squeezable or not.

One type of embodiments are puffs (poufs), pads, brushes, gloves, mitts, swabs or cotton balls.

Another type of embodiments are sponges. Sponges comprise sponges as such, foams and felts, composed of synthetic and/or natural materials.

Still another type of embodiments are bars.

For convenience of use, the applicators may have a suitable handle. Embodiments of such applicators have a pad, puff or sponge portion that preferably is resilient and a finger grip portion. One type of such applicators are those having a generally T-shaped configuration. Examples of such applicators comprise resilient discs with a small upstanding handle element.

The applicators can be made of materials which are capable of holding, adsorbing or absorbing a wax phase. Preferably, the applicator material is structured such that it is porous or absorbent in nature. The latter can be due to the chemical structure of the applicator materials or their physical arrangement or both. Examples of particular physical arrangements are porous structures, or cellular or microcellular structures.

The applicators can be made of one type of material or from different materials that can be arranged in different manners along the applicator. Small portions of one or more materials of different or equal size may be incorporated into a matrix of the same or another material. Or the applicators can be multilayered such as a stack of layers or concentric layers or they can be of one type of material. Applicator parts, either or not made of different materials can be linked together by gluing, sewing, stitching or any other technique known in the art.

In one type of embodiments the applicator comprises a core that is partially or completely wrapped in a layered material. The wrapping material may be the same or different from the material or materials used in the core.

The materials of which the applicators are made in particular are polymeric and may be both from natural and non-natural origin. There can be one or more polymeric materials that may be cross-linked or not. Optionally other non-polymeric materials such as binders, fillers, dyes and the like, may additionally be present.

The materials can be more or less inert or they can be decomposable, in particular they can be biodegradable. The materials may also be flushable. As used herein, by 'flushable' is meant that the material will pass through at least 3 meters of waste pipe in two toilet flushes.

Examples of polymeric materials of which the applicators are composed are non-natural polymers such as polyethylene, polypropylene, PET, polyamide, polyvinyl alcohol, polyurethane and the like, and natural or natural-derived polymers such as cellulose, wood pulp and the like, and mixtures of such synthetic and natural fibres or materials.

Where the applicator is in the form of a puff (pouf) it can be composed of spongy or resin foamy materials, optionally wrapped in a suitable mono- or multilayered material, which can be made of a closed or an apertured material layer or film. In other embodiments the puff is made of one or more layers of material that can be bound or glued together in the core of the puff.

Where the applicator is in the form of a bar it may be composed of wax phase material in solid state, optionally in admixture with other ingredients. Preferably, such embodiments are wrapped in a suitable layered wrapping material, which may hold the aqueous phase or the wax dispersion while the other phase is kept inside the bar as depot in the core.

The bar may be apertured, having small cavities which may hold particular ingredients.

Applicators in the form of bars may be designed such that the bar slowly decomposes or dissolves during use e.g. by body heat or by any other external factor. In particular, the bar may be composed of wax phase material which decomposes or dissolves during use, e.g. due to body heat.

If layered materials are used, these materials in themselves may be mono or multilayered, woven or non-woven. They can be made of one or of several materials. Particularly preferred layered materials are made of non-woven materials that have a web structure of fibrous or filamentous nature, in which the fibres or filaments are distributed randomly or with a certain degree of orientation, the former being obtainable by air-laying or certain wet-laying processes, the latter in other wet-laying or in carding processes. The fibres or filaments can be natural, for example wood pulp, wool cotton, linen and the like, or synthetic, for example polyvinyls, polyesters, polyolefins, polyamides and the like.

One type of layered materials is paper based, which are made almost exclusively of cellulose-based fibres. Where high wet strength or firmness of the layered material is a desired, binding materials can be added. Softness can be increased by adding additives. In another type of non-wovens the web is made mainly of staple fibre, e.g. based on cotton, wool, linen and the like.

Usually, non-woven materials for use in the applicators of the invention are made of cellulose fibres, synthetic fibres such as polyester or polypropylene or mixtures of both. Webs of increased strength can be obtained by using the so-called spunlace or hydro-entanglement technique, which does not require binding material.

One type of non-woven materials are made of a mixture of pulp and staple fibre and are available with binding materials, in particular those mentioned above, or without binding materials. In the latter instance the non-woven is preferably made by the hydro-entanglement procedure.

### The Wax Phase

In the products according to this invention, the applicator material is contacted with a wax phase. In some embodiments the applicator is contacted with a second phase which may be a polymeric phase.

Also included is the possibility to apply multiple wax phases.

The products of the invention are dry or essentially dry. Dry refers to the situation where the water content is very low, e.g. lower than 1 %. As used herein essentially dry means that the product contains limited amounts of water, e.g. less than 10 % of the total weight of the product, preferably less than 8 %, more preferably less than 5 %, still more preferably less than 2 %. It more generally means that after manufacture, no water or aqueous-based lotion is added to the applicator. As used herein a % is w/w to the total weight of the applicator with all materials incorporated therein or thereon.

The wax phase may be applied to the whole applicator, i.e. continuously, or to parts of the applicator, i.e. discontinuously. It can be applied at the surface or in the internal of the applicator. If applied at the surface, it can be present at one side or at two or more sides of the applicator.

In the instance where the wax phase is applied discontinuously, it is present at certain areas, in particular at one or more areas of the applicator. In that instance, the phase may be present as one or more forms or shapes. For example it can be present as dots or spots, lines or stripes, as geometrical figures such as squares, rectangles, circles and the like, as symbols such as letters, text, logos, figures and the like, or as trademark signs, or any other such forms, or a combination thereof. The forms or shapes may be present over the entirety of the applicator or grouped in one or more areas, for example in a corner.

In a particular embodiment, the wax phase is applied on one or more sides of the applicator in the form of stripes, dots or other forms covering the entire surface or only a part of the surface of the applicator.

Different parts of the applicator may contain different wax phases. For example the applicator may at one side contain one wax phase and at another side another wax phase.

Or the applicator may be composed of two or more parts that are linked together, each part having been treated with a different wax phase. This may result for example in applicator that at one portion has cleansing capacity and at another portion has caring capacity.

Where the applicator is in the form of a puff, a pad or a sponge it may be coated with wax phase, or the puff may have a wax phase deposed at the inner portion of the applicator. If deposed at the inner portion, the wax phase may be distributed homogeneously, meaning that is distributed over the whole inside in more or less equal quantities, or inhomogeneously.

Where the applicator is in the form of a bar or sponge, it can be wrapped into a sheet of material to which a wax phase may be applied. Furthermore, the bar or sponge material itself may contain the same or different wax phase(s). The wax phase at the outside preferably is solid while at the inside can be solid, semi-solid or liquid. The wax phase at the inner portion of the applicator may have been deposited or the applicator may have been impregnated with wax phase material in liquid form, which afterwards may solidify.

Where the applicator is in the form of a puff the wax phase may have been applied in a powdery form.

Where the applicator is in the form of a bar, it may be apertured having a plurality of cavities.

Where the applicator is in the form of a sponge it may be made of a decomposable material such as a biodegradable material. For example it can be made of dissolvable cellulose, which can be mixed with wax phase when the cellulose is still in a liquid state during the production process.

The wax phase that is applied to the applicator is such or formulated such that it is insoluble or essentially insoluble in an aqueous phase. However, in some embodiments the wax phase be mixable or soluble into an aqueous phase to a limited extend

The wax usually is solid but it can also be semi-solid. Semi-solidness can occur when the wax phase is in a transition stage between solid state and liquid state such as in a melting process, but can also be due to increased viscosity of the material that makes up the wax phase.

Semi-solidness in particular occurs with materials that have no sharp melting point, i.e. materials that have a melting range. It is also present in glass-like materials, e.g. in polymers that occur as in a glass-like state.

In particular the wax phase has a melting point or a melting range above 25 °C, for example in the range of 25 to 100°C, in particular in the range of 30 to 75°C, more in particular of 30 to 45°C, preferably in the range of 32 and 40°C. More preferably the melting temperature or melting range is above human body temperature. Most preferably the melting temperature or melting range approximates or is equal to human body temperature.

In some embodiments of this invention the wax phase may have a relatively higher melting point or range. The melting point or range may for example be higher than body temperature, e.g. higher than 40 °C, or higher than 45 °C. Upon application of such products, a more intense interaction between the wax phase and water or aqueous phase that is put on the applicator, may be required or the application of higher temperatures to promote the interaction. In the latter instance the consumer may, for example, be required to contact the product first with hot water and to then apply it or to contact it with an aqueous phase that contains agents that promote a stronger interaction with the wax phase.

As used herein the term 'melting range' refers to a temperature range that starts from the temperature at which a substance or composition loses its solid consistency up to the temperature where it becomes completely liquid. A melting range is considered to be within a defined temperature range when it overlaps with that defined temperature range, or should be considered to be above a specified temperature when the range is above said temperature.

As used herein 'ambient temperature' refers to a temperature that is in the range of about 20 to about 25 °C.

The wax phase can change to another state after application to the applicator or when being applied to the applicator during storage, or upon usage by the consumer. The wax phase may be applied to the applicator as a liquid where after it becomes semi-solid or solid. Or the wax phase may become semi-solid during usage by the consumer. This change of state may be induced by physical factors such as temperature or pressure but may also be induced by chemical factors such as particular components that cause a polymerization reaction or by a photochemical reaction.

In certain embodiments, the wax phase may be applied as two separate phases which become mixed during application on to the applicator, whereupon certain components in each phase become mixed and start to interact, e.g. in a polymerization reaction thus changing the state of the wax phase from liquid to semi-solid or solid.

Particularly preferred are the compositions of the wax phase which are solid at room temperature and which have a penetration value of 0.2 - 4 mm (measured with: Petrotester PNR 10, Mikrokonus, 5 sec., temp 20 °C).

The water content of the wax phase is low, in particular less than 10 %, preferably less than 6 %, more preferably less than 3 %, percentages being w/w relative to the total weight of the wax phase. In a particular embodiment the wax phase is water free, and will be such that it is not decomposed by water or any aqueous phase. As used herein, 'water free' means that the phase is composed of materials of low water content to which no water has been added.

In particular embodiments, multiple wax phases, i.e. wax phases of different composition, may be applied to the applicator. For example one type of wax phase is applied to one side of the applicator while another type is applied to the other. Each of these wax phases may or may not contain one or more of the ingredients mentioned hereinafter, for example one or more ingredients selected from the active ingredients, the dyes, emulsifiers, and other ingredients mentioned hereinafter. In case of various dyes, multi-colored patterns may exist, for example, each wax phase may have a different color or may be uncoloured.

The different wax phases may be applied differently at each side of the applicator. For example one side may completely be covered while the at the other side the wax phase is applied in a pattern, e.g. as stripes.

The wax phase in the products of the invention comprise dialkyl(ene) ethers or - carbonates, or dicarboxylic acids.

In a particular aspect of this invention there are provided products as specified herein wherein the wax phase essentially consists of one or more dialkyl(ene) ethers or - carbonates, or dicarboxylic acids, including mixtures thereof. The dialkyl(ene) ethers or -carbonates or dicarboxylic acids can be present in various amounts, e.g. the amounts mentioned hereinabove or hereinafter.

The dialkyl(ene) ethers or -carbonates, or dicarboxylic acids, including mixtures thereof in the composition of the wax phase allows to optimize the properties of the wax phase, in particular its sensoric properties, i.e. the products as well as the skin after the products have been applied have a less greasier feel and also a less dry skin-feel, while having excellent skin caring properties.

### Dialkyl; ethers

The dialkyl ethers are symmetric or asymmetric, straight or branch chained, saturated waxy C₁₆-C₃₀-dialkylethers, in particular C₁₆-C₂₄₋dialkylethers. More preferred are C₁₆-C₂₀-dialkylethers, and particularly preferred are distearylethers and dibehenylethers. Dialkylethers of shorter chain length can also be used such as, for example, di-n-octylcther, di-(2-ethylhexyl)-ether, laurylmethylether or octylbutylether, didodecylether. When using the latter components, the complete composition of the wax phase preferably is solid or semi-solid having the desired melting point as specified herein.

These ethers can be obtained from the appropriate fatty alcohols in the presence of an acid catalyst following art-known procedures. Typical examples are the products that are obtained by the etherification of capron alcohol, capryl alcohol, 2-ethylhexyl alcohol, capon alcohol, lauryl alcohol, myristyl alcohol, cetyl alcohol, palmoleyl alcohol, stearyl alcohol, isostearyl alcohol, elaidyl alcohol, petroselinyl alcohol, linolyl alcohol, linolenyl alcohol, oleyl alcohol, ricinus alcohol, elaeostearyl alcohol, arachidyl alcohol, gadoleylalcohol, behenyl alcohol, erucyl alcohol and brassidyl alcohol, Guerbet alcohols, as well as mixtures thereof, which, for example, are obtained by high pressure hydrogenation of technical mixtures of the methyl esters derived from fats or oils.

Of particular interest are the dialkyl ethers that are solid at 25 °C.

### Dialkyl(ene) carbonates

The dialkyl(ene) carbonates are symmetric or asymmetric, straight or branch chained, saturated or unsaturated waxy C₁₄-C₃₀-dialkyl(ene) carbonates. More preferred are C₁₆-C₂₄-dialkyl carbonates and amongst these the saturated linear C₁₆-C₂₂-dialkyl carbonates. Particularly preferred is distearyl carbonate. Also liquid dialkyl(ene) carbonates, such as, for example, dihexyl-, dioctyl-, di-(2-ethylhexyl)- or dioleylcarbonate, can be used. When using the latter components, the complete composition preferably is solid or semi-solid having the desired melting point as specified herein.

These dialkyl(ene) carbonates can be obtained by re-esterification of dimethyl- or diethylcarbonates with the corresponding hydroxy compounds following art-known procedures. Typical examples of dialkyl(ene) carbonates are re-esterification products of dimethyl- and/or diethylcarbonate with capron alcohol, capryl alcohol, 2-ethylhexyl alcohol, caprin alcohol, lauryl alcohol, myristyl alcohol, cetyl alcohol, palmoleyl alcohol, stearyl alcohol, isostearyl alcohol, elaidyl alcohol, petroselinyl alcohol, linolyl alcohol, linolenyl alcohol, oleyl alcohol, ricinus alcohol; elaeostearyl alcohol, arachidyl alcohol, gadoleyl alcohol, behenyl alcohol, erucyl alcohol and brassidyl alcohol, Guerbet alcohols, as well as technical mixtures thereof, that can be obtained by hydratation of methyl esters derived from suitable oils or fats or oil or fat fractions.

Of particular interest are those dialkyl(ene) carbonates that are solid at 25 °C.

### Dicarboxylic acids

Dicarboxylic acids that can be used are, for example, C₉-C₃₄-dicarbonic acids. Of particular interest are those discarboxylic acids that are solid at 25 °C.

The total amount of one or more of the dialkyl ethers, dialkyl carbonates, and dicarbonic acids present in the wax phase, relative to the total weight amount of the wax phase, is in the range of 1 - 30 % (w/w), preferably of 1 - 20 % (w/w) more preferably from 1 -10 % (w/w).

### Additional Waxes

The wax phase may comprise additional waxes. As used herein, the term 'wax' refers to oil soluble materials that have a waxy consistency and have a melting point or melting range of above ambient temperature, in particular above 25 °C. Waxes are materials that have a solid to semi-solid (creamy) consistency, are crystalline or not, being of relatively low viscosity a little above their liquefying point. Waxes can be composed of one or more components, synthetic as well as natural, and can in principle be composed of or comprise any oil soluble material having a waxy consistency, including mixtures thereof.

Waxes which can be used may be synthetic or natural waxes, as well as other oil soluble materials that have a waxy consistency. Waxes also encompass materials such as oils or fats of natural or synthetic origin, and waxy components such as higher alkanols (in particular fatty alcohols), higher alkanediols (in particular hydroxy fatty alcohols), carboxylic acids (in particular fatty acids), dialkyl(ene)ethers, dialkyl(ene) carbonates, dicarboxylic acids and the like components.

Natural waxes comprise waxes from vegetal origin, such as purcelline, shea butter, cocoa butter, Japan wax, esparto gras wax, cork wax, Guaruma wax, rice shoot wax, Ouricury wax, montan wax, sunflower wax, ceresine wax, sugar cane wax, carnauba wax, candelilla wax, lanolin, fruit-derived waxes, such as orange wax, lemon wax, grapefruit wax and bayberry wax, and the like, and of animal origin such as beeswax, woolwax, spermateci and bear fat, shellac wax, and the like. Natural waxes further comprise mineral waxes such as ceresine and ozokerite waxes. Synthetic waxes comprise petroleum-based waxes such as paraffin, vaseline, petrolatum, micro wax. Further synthetic waxes are polyalkylene and polyethyleneglycol waxes, e.g. polyethylene wax; waxes based on chlorinated naphtalenes such as 'Halowax', synthetic hydrocarbon waxes, and the like, including mixtures thereof. Further waxes are chemically modified waxes, in particular hardened or hydrogenated waxes such as, for example, Montan-ester waxes, Sasol waxes and hydrogenated jojoba waxes. Preferred among these natural waxes are waxes from vegetal origin.

Other wax components can be certain fats (including mono-, di- and triglycerides and fatty acid alkylesters), fatty alcohols, fatty acids, including substituted fatty acids (in particular hydroxy substituted fatty acids, for example, 12-hydroxystearic acid), dialkyl(ene)ethers, dialkyl(ene) carbonates, dicarboxylic acids (in particular the C₁₆-C₄₀-dialkylesters of dicarboxylic acids, e.g. the C₁₆-C₄₀-alkyl stearates, C₁₈-C₃₈-alkylhydroxystearyl stearates or C₂₀-C₄₀-alkyl erucates) and hydroxy fatty alcohols that comply with the definition of 'wax' as outlined herein. Any of these components may contain homologous components that are liquid, as long as the total composition making up the wax phase has a waxy constituency. For example, waxy fats may contain oils, waxy fatty alcohols may contain liquid fatty alcohols, etc., in such amount that the total composition has a waxy consistency and in particular has the melting point or melting range specified above.

Still further wax components are selected from the group of aromatic carbonic acids, tricarboxylic acids, or from the group of lactides of long-chained hydroxycarbonic acids. Myristyl lactate is particularly attractive for skin treatment, because of its binding capacity to the skin.

Further wax components that can be used are C₃₀-C₅₀-alkyl bees wax; tri-C₁₆-C₄₀-alkyl citrates, e.g. tristearyl citrate, triisostearyl citrate, trilauryl citrate; ethyleneglycol difatty acid esters, in particular the ethylene glycol di-C₁₂-C₃₀-fatty acid esters, e.g.ethyleneglycol dipalmitate, ethyleneglycol distearate, ethyleneglycol di(12-hydroxystearate). As further useful components there can be mentioned silicone waxes.

The wax phase may also comprise mixtures of waxes and oils.

The total amount of additional waxes in the wax phase may be up to 50 %, in particular up to 30 %, more in particular up to 20 %, w/w of the total amount of components making up the wax phase.

### Oils and Fats

The wax phase may further contain fats and oils, the latter to such an extent that the wax phase remains solid or semi-solid at ambient temperature, in particular at 20 °C or at 25 °C.

Oils or fats which can be used in the wax phase comprise natural oils or fats, or natural oil or fat derivatives, in particular of vegetable origin. Examples are almond oil, soybean oil, sunflower oil, safflower oil, corn oil, kernel oil, canola oil, borage oil, evening primrose oil, grapeseed oil, wheat germ oil, avocado oil, jojoba oil, sesame oil, walnut oil, linseed oil, palm oil, olive oil, macadamia oil, castor oil, rapeseed oil, peanut oil, coconut oil, turnip seed oil, and the hardened derivatives thereof. The latter are obtained by hydrogenation of fats or oils. Preferred are hardened oils or fats from vegetal origin, e.g. hardened castor oil, peanut oil, soya oil, turnip seed oil, cotton seed oil, sunflower oil, palm oil, kernel oil, linseed oil, almond oil, corn oil, olive oil, sesame oil, cocoa butter, shea butter and coconut oil.

Said hardened fats or oils have the additional advantage of increasing the constituency of the wax phase.

The wax phase may further comprise fatty components isolated from these natural oils, i.e. pure triglycerides or mixtures thereof, or the latter components having been prepared chemically. These so-called trigycerides (or triacyl glycerines) are esters of glycerines with fatty acids or fatty acid mixtures, for example so called technical mixtures obtained by hydrolysis from fractions of oils or fats, or by fractioning fatty acid mixtures after hydrolysis. The triglycerides may also be obtained chemically by synthesis.

The fatty acids in said triglycerides may be saturated or unsaturated, straight or branch chained, substituted or unsubstituted. Preferred triglycerides are those glycerines esters derived from fatty acids, either saturated or unsaturated, having from 10 to 60, in particular from 12 to 36, more particularly from 12 to 24, preferably from 16 to 20 carbon atoms. Preferred such fatty acids are, for example, palmitic, palmic, oleic, lauric, myristic, stearic, hydroxystearic, behenic acid, or mixtures thereof. Within this group the triglycerides derived from saturated fatty acids are of particular interest.

Of specific interest are glyceryl tristearate, also referred to as stearin, glycerine tribehenate, glycerine tripalmitate, glycerine trilaurate, glycerine trioleate, glycerine trimyristate.

The wax phase may also contain mono- or diglycerides, optionally in a mixture with the fats and oils mentioned herein, in particular with triglycerides. The mono- or diglycerides for use in the wax phase are derived from saturated or unsaturated, linear or branch chained, substituted or unsubstituted fatty acids or fatty acid mixtures. Also in this instance the melting point or melting range of the wax phase preferably is as mentioned above, in particular is above ambient temperature, more in particular is in the range of 32 °C to 40 °C. Particular mono- or diglycerides are mono- or di-C₁₂₋₂₄ fatty acid glycerides, specifically mono- or di-C₁₆₋₂₀ fatty acid glycerides, for example glyceryl monostearate, glyceryl distearate. Mixtures of mono-, di- and, optionally, triglycerides can be derived from fractions of fatty acids. An example of such mixture for use as a component of the wax phase is a mixture of C₁₂₋₁₈ mono-, di- and triglycerides.

In a preferred embodiment according to the present invention the wax phase contains one or more fatty acid glycerides selected from the mono-, di- or triesters from glycerine, or a mixture thereof.

The amount of said fatty ester glycerides in the wax phase may be up to 50 % and more preferably up to 40 % (w/w), relative to the total quantity of the wax phase.

Mixed esters as well as mixtures of mono-, di- and triglycerides are of particular interest because of their low propensity to crystallize and their capacity to improve the consistency of the formulation making up the wax phase.

The wax phase may also comprise alkyl esters of fatty acids, wherein the alkyl group has from 1 to 30 carbon atoms, preferably from 12 to 24 carbon atoms. The fatty acids in said alkyl esters in particular are C₁₂₋₃₀ fatty acids, more in particular C₁₂₋₂₀ fatty acids. The alkyl groups in said esters preferably are derived from fatty alcohols as well as of mixtures thereof, which, for example, are obtained by high pressure hydrogenation of technical mixtures of the methyl esters derived from fats or oils.

Preferred are the alkyl esters of C₁₆₋₂₄ fatty acids, more preferably from C₁₆₋₁₈ fatty acids, and C₁₋₃₀ fatty alcohols, preferably C₈₋₂₄ fatty alcohols, more preferably C₁₂₋₂₀ fatty alcohols.

Of particular interest in this regard are, e.g. stearyl stearate, palmityl stearate, stearyl behenate, cetyl stearate, cetyl behenate, cetyl palmitate, cetearyl behenate, behenyl behenate, stearyl heptanoate, stearyl octanoate, myristyl myristate, myristyl isostearate, myristyl oleate, cetyl isostearate, cetyl oleate, stearyl isostearate, stearyl oleate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl isostearate, behenyl oleate, erucyl isostearate.

Of further interest are esters of linear C₆-C₂₂-fatty acids with branched alcohols, in particular 2-ethylhexanol, esters of branched C₆-C₂₂-fatty acids with linear alcohols, esters of C₁₈-C₃₈-alkylhydroxycarbonic acids with linear or branched C₆-C₂₂-fatty alcohols, esters of linear and/or branched fatty acids with poly-alcohols (e.g. propylene glycol, dimerdiol or trimertriol) and/or Guerbet alcohols, as well as esters of C₆-C₂₂-fatty alcohols and/or Guerbet alcohols with aromatic carbonic acids, in particular benzoic acid, esters of C₂-C₁₂-dicarbonic acids with linear or branched C₁-C₂₂ -alcohols (e.g. dioctyl malate) or C₂-C₁₀ -polyoles having 2 to 6 hydroxy groups.

Preferred fats comprise the triglycerides, in particular those derived from fatty acids having from about 12 to about 24 carbon atoms, in particular those having from about 12 to about 20 carbon atoms, more in particular those having from about 16 to about 20 carbon atoms. These fatty acids may be unsaturated or, which is preferred, saturated.

Particularly preferred are glycerides derived from oleic, stearic, myristic or lauric acid, or from fatty acid mixtures derived from natural oils such as coco-acids. Examples of preferred fats are cocoglycerides, glyceryl stearate, glyceryl laurate, and the like.

Further preferred fats comprise hydrogenated natural oils such as hydrogenated castor oil, hydrogenated palm oil and the like.

The wax phase.may also comprise oily components, i.e. non water-mixable components that are liquid at 20 °C. These can be e.g. glycerides, hydrocarbons, silicon oils, ester oils and the like, as well as mixtures thereof. The total quantity of these oily components in the total composition of the wax phase preferably will be such that the wax phase is solid at room temperature, or that it has a melting point or range that is as specified hereinabove. The oily components will typically be present in quantities of less than 40 % (w/w), in particular less than 20 % (w/w), or further in particular 1-15 % (w/w), more in particular from 2 - 10 % (w/w) relative to the total weight of the wax phase.

The oily components can be any of the oils mentioned hereinabove as 'oils and fats', more in particular the mono-, di- and triglycerides mentioned hereinabove, that are liquid at 20 °C. The oily components can further be fatty acids and fatty alcohols, described in this specification that are liquid at 20 °C.

Further oily components which can be used in the wax phase comprise silicone oils, mineral and paraffin oils and synthetic oils, either aliphatic or aromatic, as well as mixtures thereof. Examples of such oils are squalane, squalene, isohexadecane, isoeicosane, polydecene, and also oils of the group of dialkylcyclohexanes.

The wax phase may further contain silicone oils, volatile or not, such as, for example, cyclic silicones, dialkyl- or alkylarylsiloxanes, e.g., cyclomethicone, dimethyl polysiloxane (dimethicone) and methylphenyl polysiloxane, as well as the alkoxylated and quaternized derivatives thereof. Appropriate non-volatile silicone oils are e.g. longer chain polyalkylsiloxanes and polyalkylarylsiloxanes, and also polyethersiloxane-copolymers.

### Fatty alcohols

The wax phase may also comprise fatty alcohols. Fatty alcohols that can be used are, for example, C₁₂-C₅₀-fatty alcohols, in particular the C₁₂-C₂₄-fatty alcohols, that are derived from natural fats, oils or waxes such as, for example, myristyl alcohol, 1-pentadecanol, cetylalcohol, 1-heptadecanol, stearyl alcohol, 1-nonadecanol, arachidyl alcohol, 1-heneicosanol, behenyl alcohol, brassidyl alcohol, lignoceryl alcohol, ceryl alcohol or myricyl alcohol as well as Guerbet alcohols. Preferred for use in the present invention are saturated, straight or branch chained fatty alcohols. However also unsaturated, straight or branch chained alcohols can be used, optionally in a mixture with saturated alcohols. Preferably the alcohols will be selected such that the melting point of the mixture is as referred to hereinabove and more in particular is in the range of 32 to 40 °C.

Mixtures of fatty alcohols can evidently also be used, including fatty alcohol fractions obtained from the reduction of the corresponding fatty acid fractions derived from naturally occuring oils or fats such as, for example, almond oil, soybean oil, sunflower oil, safflower oil, corn oil, canola oil, borage oil, evening primrose oil, grapeseed oil, wheat germ oil, avocado oil, jojoba oil, sesame oil, walnut oil, linseed oil, palm oil, olive oil, castor oil, macadamia oil, rapeseed oil, peanut oil, coconut oil, and turnip seed oil.

Synthetic alcohols can also be used such as, for example, the linear fatty alcohols of an even number of carbon atoms resulting from the Ziegler-synthesis (Alfole^{®}) or the partially branched alcohols resulting from the Oxo synthesis (Dobanole^{®}).

The use of fatty alcohols advantageously results in the wax phase having a drier, i.e. less greasy, skin feel, compared to components such as triglycerides.

The total amount of fatty alcohols in the wax phase may vary and depends on the desired properties of the wax phase. The total amount of the fatty alcohols present in the wax phase may be in the range of 0 - 40 % (w/w), preferably of 1 - 30 % (w/w), more preferably of 1 - 20 % (w/w), still more preferably from 1 -10 % (w/w) of the total amount of components making up the wax phase.

### Fatty acids

The wax phase may also contain C₁₄-C₄₀-fatty acids, including mixtures thereof. Of particular interest are the C₁₆-C₃₀-fatty acids. These comprise, for example, myristic-, pentadecanoic-, palmitic-, margaric-, stearic-, nonadecanoic-, arachic-, behenic-, lignoceric-, cerotic-, melissic-, erucaic-, elaeostearic-, oleic-, lonolenic-, lauric acid as well as substituted fatty acids, e.g. hydroxy-substituted fatty acids such as, for example, 12-hydroxystearic acid, and the amides or monoethanolamides of these fatty acids.

The total amount of the C₁₄-C₄₀-fatty acids present in the wax phase, relative to the total weight amount of the wax phase, may be in the range of 0 - 30 % (w/w), preferably of 1 - 20 % (w/w), more preferably from 1 -10 % (w/w) relative to the total amount of components making up the wax phase.

### Further components

The compositions of the wax phase may contain further components, which may be of waxy nature or otherwise. The use of these further components allows to influence the sensorical properties as well as the stability of the compositions, in particular after application to the applicator material and more in particular when in contact with water or an aqueous phase. The other components may also be added to influence consistency, feel and appearance. These components will generally be insoluble or poorly soluble in water. Water-soluble components can also be included, typically in combination with a solubilizing or emulsifying agent and some water.

Examples of further components are superfatting agents, thickeners, polymers, active ingredients, film forming agents, UV-filters, anti-oxidants, hydrotropic agents, preservatives, insect repellents, self-tanning agents, solubilizers, perfume oils, dyestuffs, consistency agents, and the like.

Appropriate cationic polymers are for example cationic cellulose derivatives , e.g. quaternized hydroxyethyl cellulose (commercialized under the trade name Polymer JR 400^{®} by Amerchol), cationic starches, copolymers of diallylammonium salts and acrylamides, quaternized vinylpyrrolidone/vinylimidazole-polymers (for example Luviquat^{®} of BASF), condensation products of polyglycols and amines, quaternized collagen polypeptides, such as, for example, lauryldimonium hydroxypropyl hydrolyzed collagen (Lamequat^{®}L/Grünau), quaternized wheat polypeptides, polyethylene imines, cationic silicone polymers, e.g. amodimethicone, copolymers of adipinic acid and dimethylaminohydroxypropyldiethylenetriamine (Cartaretine^{®}/Sandoz), copolymers of acryl acid with dimethyldiallylammonium-chloride (Merquat^{®} 550/Chemviron), polyaminopolyamides, cationic chitine derivatives such as, for example, quaternized chitosans, optionally dispersed in microcristalline form, condensation products derived from dihalogenalkylenes, such as, for example dibromobutane with bis-dialkylamines, e.g. bis-dimethylamino-1,3-propane, cationic guar-gum derivatives, such as, for example, Jaguar^{®} CBS, Jaguar^{®} C-17, Jaguar^{®} C-16 from Celanese, quaternized ammonium salt-polymers, e.g. Mirapol^{®} A-15, Mirapol^{®} AD-1, Mirapol^{®} AZ-1 from Miranol.

Anionic, zwitterionic, amphoteric and nonionic polymers that can be used are, for example, vinylacetate/crotonic acid-copolymers, vinylpyrrolidone/vinylacrylate-copolymers, vinylacetate/butylmaleate/ isobornylacrylate-copolymers, methylvinylether/maleic acid anhydride-copolymers and their esters, which are not cross-linked and with polyoles linked polyacrylacids which are cross-linked, acrylamidopropyl trimethylammonium chloride/ acrylate-copolymers, octylacrylamide/methylmethacrylate/tert.butylaminoethylmethacrylate/2-hydroxypropylmethacrylate-copolymers, polyvinylpyrrolidone, vinylpyrrolidone/vinylacetate-copolymers, vinylpyrrolidone/ dimethylaminoethylmethacrylate/vinyl caprolactam-terpolymers as well as optionally derivatized cellulose ethers and silicones.

The wax phase may further contain suitable anti-oxidants, and powders or powdered ingredients.

The wax phase may further contain disintegrating agents, which are agents that cause a disintegration of the physical integrity of the wax phase. The disintegration may be in parts or on the whole of the wax phase. The disintegrating agents may be mixed or dissolved into parts or the whole of the wax phase. The disintegrating agents may be mixed continuously in the wax phase or discontinuously, e.g. at the top side of the wax phase, e.g. where the wax phase is applied as a layer, at the top of that layer or in the top portion of that layer.

Suitable disintegrating agents are agents that are subject to physical or chemical interactions either by auto-interaction or by interaction between two agents. This results in a physical or chemical interaction with the wax phase. One type of disintegrating agents are those that release a gas e.g. by decomposition or by chemical reaction between two components. An example of a disintegrating agent is a solid mixture of a bicarbonate and an acid such as sodium or potassium bicarbonate with a suitable organic acid, e.g. citric acid. Upon contact with water, e.g. upon contact with an aqueous phase, the disintegrating components will interact and liberate carbon dioxide which physically alters the wax phase. Such physical alteration may, for example, cause the wax phase to become homogeneously distributed on the applicator. This may positively influence the interaction between any aqueous and wax phases, which in turn may have a positive effect on the transfer to the skin of materials, e.g. active ingredients, in these phases.

The wax phase may further contain components that are subject to a polymerization reaction either during or after application on the applicator material. Examples of such components are oligomers that during or after application on the applicator continue to polymerize with monomers or other oligomers. Other examples are agents that cause netting or co-polymerisation. There can also be agents that inhibit polymerization for a specific period of time. Alternatively there can be agents that accelerate polymerization e.g. under influence of external factors such as heat, light or pressure.

In one type of embodiments, the wax phase contains monomers or oligomers that can be caused to polymerize or co-polymerize under the influence of an external factor, an example of the latter being light. The wax phase is applied to the applicator and during the application process the wax phase is subjected to light radiation whereupon polymerization occurs. Alternatively, the wax phase may be subjected to light radiation after it having been applied to the applicator.

The wax phase may further contain dyes that upon usage of the product change color due to a change of temperature or pressure. This will give the consumer a level of comfort and trust that the product delivers the wax phase to the skin, or in case of a wax phase containing active ingredients that the latter are delivered onto the skin.

The wax phase may further contain dye-precursors, i.e. agents that become dyed upon influence of physical or chemical factors. In particular embodiments the wax phase may contain dye-precursors which react with certain agents that are present in an aqueous phase so as to form a dye. Similarly, the dye-precursors may be present in the aqueous phase and become transferred into dyes upon interaction with certain chemicals incorporated into the wax phase.

The wax phase can also be formulated to or into beads. Particularly such beads are polymeric beads wherein the wax phase is entrapped in whatever form. The terms 'beads' or 'polymeric beads' are meant to comprise any form of discrete, free-flowing powders, beads or capsules which envelope, coat or contain a wax phase in a mono- or polymeric matrix or capsule. These terms also encompass powders, beads or capsules wherein the mono- or polymeric matrix itself is a wax phase. These terms are also meant to include porous beads or 'microsponges' and 'microcapsules', the latter being beads of smaller size. The beads may be coated with a suitable coating material that protects the interior of the bead or controls the release of the wax phase entrapped therein. The coating on the bead itself may contain a wax phase. In the latter instance, the coating is layed on an inert core or on a core containing wax phase and/or other ingredients.

Formulation of a wax phase in beads may be done for protecting the wax phase from external factors that may impact its integrity. However, it is mostly done for allowing controlled release of the wax phase.

A particular type of beads are small beads or capsules, having an average diameter which is in the micrometer range, although the average diameter can be as small as even 200 nm.

This type of capsules can be liposome-based, made for example of phospholipids such as lecithin, phosphatidyl ethanolamine, phosphatidyl serine, phosphatidic acid and the like. This type of capsules also can be made of starch, cellulose, porous gelatin and the like.

The capsules or beads can also be relatively larger, having average sizes in the mm or 0.1 mm range. This type of capsules or beads can be made of materials such as agar, glycolic acid polymers, and further components such as water, mineral oils, glycerin. They may contain further ingredients such as preservatives, dye(s), and the like. Another type of beads or microcapsules are microsponges. These are materials sized from about 5 to about 300 µm (average diameter) having a large inner surface. These are obtained by polymerization of particular monomers. Wax phase material can be entrapped therein either during this polymerization process or afterwards. Microsponge-based carriers may be used to protect the wax phase entrapped therein or for controlled release purposes.

The capsules may optionally contain one or more suitable disintegrating agents, in particular those mentioned in this specification. Upon contact with the appropriate external factor, the disintegrating agents will cause the capsules to break open thus allowing release of the wax phase entrapped therein.

The capsules can be incorporated into another wax phase, or in both. They can also be applied to the applicator prior to the introduction of the wax phase. They can even be introduced during the manufacturing process of the applicator itself.

Release of the wax phase from the beads or capsules can be the result of the rupture of the coating or from the matrix. This may be the result of physical factors such as pressure, strain or by shearing forces upon use of the applicator product, e.g. by rubbing the product to the skin or to a surface. Release of the wax phase may be due to the semi-permeable or porous nature of the bead or its coating or due to external factors such as contact with liquid media that cause the wax phase to become extracted, or to dissolve or disintegrate the bead or its coating, or by temperature effects. The capsules can also be disintegrated under influence of certain chemicals, in particular by disintegrating agents incorporated into the capsules. Particular embodiments of the latter are capsules containing suitable amounts of bicarbonate and an organic acid which, upon contact with water, e.g. upon contact with an aqueous phase when using the applicator product, cause the capsules to disintegrate.

The beads or capsules can be made according to methodologies generally known in the art, for example by emulsion polymerisation.

The beads or capsules may be applied to any portion of the applicator but preferably they are concentrated at the surface or in the upper surface portion of the applicator. This allows maximal transfer of the wax phase to the skin or to the surface to which the product is applied.

The beads or capsules can be applied to the applicator in dry form by dusting, sifting, spraying and the like methods. They can also be printed or roll-coated in the form of a suitable liquid or paste. They can also be mixed with a suitable liquid, which can be a solvent that is inert towards the beads, or water, or an aqueous phase, and sprayed onto the applicator.

### Preferred compositions

In preferred embodiments, the composition of the wax phase has a melting point or melting range of above 25 °C, preferably in the range of 30 to 45 °C, more preferably in the range of 32 to 40 °C.

The water content of the preferred compositions of the wax phase is low, e.g. lower than 10 %, preferably lower than 6 %, more preferably lower than 3 % w/w relative to the total weight of the wax phase. In particular, the preferred compositions will be water free.

The wax phase may also contain liquid dialkyl(ene) ethers, dialkyl(ene) carbonates, dicarbonic acids or hydroxy fatty alcohols, however preferably in such amounts that the melting point or range of the total composition of the wax phase does not exceed 25 °C, and more preferably is within the temperature ranges mentioned above.

In particularly preferred embodiments, the products of this invention have a wax phase containing:
(a) at least 1 - 50 % (w/w), in particular at least 1 - 30 % of an oily or waxy component selected from C₁₄-C₃₀-dialkyl ethers, C₁₄-C₃₀-dialkyl carbonates, C₄-C₃₄-dicarbonic acids or C₁₂-C₃₀-hydroxyfatty alcohols or mixtures thereof
(b) 0 - 5 % (w/w), in particular from 0.1 - 5 % (w/w) of at least one active ingredient
(c) 0 - 10 % (w/w), in particular from 1 - 10 % (w/w), of at least one oil
(d) 0 - 10 % (w/w), and in particular from 0.1 - 10 % (w/w) of at least one emulsifier
(e) 0 - 90 % (w/w), and in particular from 5 - 90 % (w/w), of further waxy components
(f) 0 - 5 % (w/w), and in particular 0 - 3 % (w/w), water

### Application of the wax phase

The wax phase may be applied to the applicator in various ways. Preferably the wax phase is applied at the surface or at the surface portion of the applicator, on one or on several sides.

The wax phase can be applied evenly or non-evenly to the applicator, non-evenly meaning that the distribution of the amount of the wax phase varies over the area of the applicator, i.e. some areas of the applicator can have greater or lesser amounts of the wax phase. Preferably the wax phase is evenly applied to the area of the applicator.

The wax phase can be applied discontinuously or continuously to one or several sides of the applicator, or it may even be applied as a complete covering of one or several surfaces of the applicator.

The wax phase preferably is applied in a discontinuous pattern, to one or several sides of the applicator. To this purpose the wax phase is applied in a predetermined, controlled manner to specific areas of the applicator. A discontinuous pattern is one in which the wax phase has been applied to distinct regions separated by regions of the applicator which are free of the wax phase. The wax phase in that instance is applied to defined parts or regions of the applicator which may take a variety of forms. The wax phase may in particular be applied as described above more generally for the application of both phases. Particular forms in which the wax phase may be applied are, e.g. stripes, dots or spots, geometric configurations, either of regular or irregular shape, for example circles, ellipses, squares, rectangles and the like, logos, text, letters or any other non-continuous pattern, including the patterns described hereinabove more generally for the application of the wax phase.

Discontinuous patterns also comprise essentially networks of larger patterns of the wax phase. In a preferred embodiment, the wax phase is present as discrete stripes which can be disposed discontinuously, i.e. interrupted, or preferably continuous over the whole surface of the applicator. The stripes may also form a pattern of discrete segments which collectively comprise a stripe or they may have a repetitive pattern such as a sinusoidal shape or wave-like and the like pattern. If waving stripes are selected, preferably the stripes are in phase, so that parallelism is maintained and each stripe remains equally spaced from the adjacent stripes.

The stripes are preferably oriented in the machine direction, for ease of manufacture.

In certain embodiments more than one wax phase may be applied to one or several sides of the applicator. For example one wax phase may be applied on the entire surface or part of the surface of one side of the applicator, whereas another wax phase is applied on the entire other side or only partly, either with the same or another pattern than the other wax phase. Particular such embodiments are those having two different wax phases on the same side e.g. in parallel stripes or other patterns with the same or different colors.

In particular embodiments, not more than half of the surface of the applicator, either on one side or, which is preferred, on several sides is carrying or covered by the wax phase. In a preferred embodiment, the wax phase is present at the surface on several sides, covering not more than 50 % of the applicator's surface, in particular covering not more than 35 % or not more than 25 % of the surface. In a particularly preferred embodiment, the wax phase is present as stripes, in particular as parallel stripes running in parallel with the side of the applicator, covering not more than half or, more in particular 25 % of the surface. In another particularly preferred embodiment, the wax phase is present as dots, equally spread over the entire surface of the applicator, covering not more than 50 % of the surface.

Some embodiments have more or less regularly shaped dots, other embodiments have circle-shaped dots, others have ellipsoids, while still others have mixed patterns, e.g. combinations of circles and ellipsoids, of regularly shaped dots and circles and the like.

In case of stripes, the width thereof which preferably is between 1 to 10 mm, more preferably between 3 to 7 mm. In case of dots, round shapes are preferred, e.g. circles or ellipsoids, with an average diameter between 1 to 10 mm, more preferably between 3 to 7 mm. There can be stripes with different widths on one product, and there can be dots of different size on one product. An example of an embodiment of the latter is a applicator with circles of a certain size and ellipses of a different size, or of circles with different sizes.

The wax phase may be colorless or colored, i.e. mono- or multi-colored. Multi-colored patterns are obtained by applying several wax phases that have been dyed differently. A colored wax phase will alert the user of the fact that the applicator is covered by a special material that contains an active ingredient or it may also make the product aesthetically attractive.

In another embodiment the applicator itself is colored, either at several sides or only at one side, over the complete surface or only at parts. If the color is present only at parts of the applicator it preferably will take the shapes and forms described in connection with the patterns that the wax phase may take. In other embodiments only the space between the surface portions at which the wax phase is applied is colored thus leaving the areas of the wax phase uncolored. In this way, the patterns of the wax phase will appear as uncolored patterns.

A preferred pattern for coloring the applicator is in stripes. Examples of such embodiments are those wherein the colored stripes or the area between the colored stripes are covered with wax phase. In the former instance the wax phase stripes are colored, in the latter they are uncolored.

The wax phase, which itself can be colored or uncolored, may be applied to the colored applicator in a number of different ways. In case of applicators having a completely colored surface, the wax phase can be applied over the whole surface thus resulting in a different or altered color, e.g. a more pale color where the wax phase is white or opaque. The wax phase can also be applied in certain patterns, thus resulting in multicolored products or where the wax phase is white or opaque in products with mono-colored patterns. Also in this instance, the preferred pattern is in stripes.

In still a further embodiment, the applicator is colored in certain patterns and the wax phase is applied on these patterns or part of these patterns. Also in this instance the wax phase may be colored or uncolored, i.e. white, opaque or transparent. In case the lipid phase is white or opaque its thickness may be selected such that the color of the underlying section of the applicator is visible thus giving the consumer the impression that a lipid phase containing a particular ingredient is present.

The wax phase is typically applied in an amount of from about 3 to about 40 g/m², preferably from about 10 to about 20 g/m², either on one side or, preferably, on several sides of the applicator. Or, alternatively, the wax phase is applied in an amount of about 0.06 g to about 0.8 g per gram of substrate, preferably from about 0.20 g to about 0.40 g per gram of dry substrate.

The wax phase can be applied to the applicator by any method that can be used to contact or impregnate a liquid or molten lipid material to or in a applicator. The wax phase may be applied by bathing the applicator into a liquid wax phase. Where the latter is solid or semi-solid at room temperature, it is liquefied by melting or dissolving into a suitable solvent which is evaporated afterwards.

The wax phase can also be applied by any method that allows coating of the lipid material onto the surface of the applicator. As used herein the term 'coating' refers to printing, covering, overlaying, finishing, spraying, extruding, laminating or any other method of applying the phase to the surface of the applicator.

A particular coating technique is extrusion wherein the composition is forced through tubes in contact with the applicator while the applicator passes across the tubes. A preferred technique comprises contacting the applicators with a heated head equipped with a slit blade, i.e. a blade having cut-out areas, wherefrom the wax phase, in molten state, is extruded. Another preferred coating technique involves the so-called hot melt process which comprises spraying the liquefied wax phase from a heated spraying head or nozzle. Another application technique involves spraying or drippling the composition on a rotating surface such as calender roll that then transfers the composition to the surface of the applicator.

Still another technique is based on traditional printing technologies which comprise, for example, screen printing, roller printing and gravure printing. In general, printing comprises techniques wherein a rotating surface is provided with elevations (by engraving, embossing or similar techniques) and the elevations are contacted with the liquefied wax phase, e.g. by running it through a bath with liquefied phase one, and thus printed on the applicator. Another technique to apply the wax phase is by using a screen printing procedure where the molten wax phase is introduced into a rotating roll and squeezed through a metal screen that covers the roll. This leads, depending on the design of the screen, to a defined pattern on the applicator like stripes, dots, squares, circles and the like, or even logos and text.

A further technique to apply the wax phase onto the applicator is by roller-ball application which comprises contacting a ball which is in direct contact with the applicator, with wax phase in liquid state and transferring it through a rolling movement onto the applicator. Depending on the desired pattern of the wax phase on the applicator, there can be several of such roller-ball applicators mounted next to one another, or after one another. They may contain the same or different wax phases.

The wax phase may also be applied by high-pressure coating. In one type of execution of this procedure the wax phase is applied via extrusion through appropriate nozzles, under high pressure. Specially shaped nozzles may be used resulting in particular patterns. For example there can be nozzles that result in circles, stars, squares, or other geometric shapes or even irregularly shaped patterns.

The wax phase may also be applied by a combination of these application techniques.

The wax phase may also be applied to the applicator in dry form as particles or as powder. In one type of embodiments the wax phase is applied as beads or small capsules, e.g. by drippling or screen printing. After application the particles may be caused to melt thereby forming small dots in or on the applicator.

The wax phase preferably is applied in liquid form, e.g. in its molten form.

The wax phase may be applied in liquid form while being in admixture with water, which can be colored or uncolored and which is removed after application to result in a dry or essentially dry product. 'In liquid form' in this context means that the wax phase is liquid in itself or is liquefied by heating, e.g. by heating in the water in which it is applied. The wax phase is kept liquid all along the process. In the instance of a solid wax phase, it is only allowed to solidify after removal of the water that has been added. In one embodiment, the wax phase is mixed with hot water whereupon the lipid/water mixture is applied to the applicator. The water is subsequently evaporated which may be accomplished by a variety of means, e.g. by simply allowing the water to evaporate, by passing the applicator over one or more heated rolls, thus forcing the water to evaporate, by applying dry air, either heated or not, by applying reduced pressure.

In the execution where the water is colored, it will diffuse into the applicator and after its evaporation leave the applicator colored. The wax phase that has been applied in this type of execution may be uncolored, in which case it will appear as white or lighter areas. Or the wax phase may be colored which will result in a multi-colored product. In another execution, the wax phase in this process is colored and uncolored water is used resulting in products wherein the wax phase areas are colored and the areas and the other areas are uncolored. The thus obtained products may subsequently be treated with aqueous phase which may be colored or not, resulting in products with even more color combinations.

In one type of embodiments, the wax phase is applied as a layer on the applicator, either continuously or discontinuously, at one or several sides of the applicator and this layer is dotted with particles of wax phase material that are punched into the surface of the lipid layer by application of pressure. The material of the dots may be the same or different as that of the lipid layer.

The wax phase preferably is applied in such manner that it will remain on the applicator surface during the manufacturing process and storage. This can conveniently be accomplished by applying the wax phase above its melting temperature, e.g. by spraying or coating it when molten to the surface of the applicator and subsequently allowing it to cool below its melting point so that the phase solidifies.

The wax phase preferably is applied such that it is present at the surface of the applicator because of its physical location in that instance, the wax phase is readily available to be spread onto the skin during usage. As a result, the effectiveness with which the wax phase is transferred to the skin during use, the availability and therefore the effectiveness of any active ingredients incorporated therein is increased compared to products where the active agent is simply incorporated into a single continuously applied phase.

In preferred embodiments, the melting point or range of the wax phase is above 25 °C, or within the temperature ranges specified above, because this allows to apply the wax phase in liquid (molten) state to the applicator, and subsequently, after it having been cooled, to be present in solid state on the applicator. In preferred embodiments, the wax phase forms a weak non-brittle film on the applicator. Applicators that have been treated this way are particularly stable, in particular during storing, essentially because mixing of the two phases is avoided. Additionally such applicators will allow the wax phase to melt upon contact with the skin, thus allowing a local mixing or emulsification of both phases.

In some embodiments of this invention the products may contain two or more wax phases with different stability towards water or an aqueous phases. This allows one phase to interact more quickly with water or an aqueous phase than the other. This may find application in products where a gradual of active ingredient is desired or the release of a sequence of two or more active ingredients.

### Wetting the Product

During or prior to usage the products of this invention can be wetted with water or suitable aqueous phases. The latter can be any of the art-known aqueous based formulations used to impregnate applicators. Beside water the aqueous phase may also contain further ingredients or additives such as surfactants, emulsifiers, consistency factors, conditioners, moisturizers, thickeners, preservatives, active ingredients, in particular dermatologically active ingredients, fragrances and the like. Active ingredients as mentioned herein comprise, for example, anti-inflammatories, anti-bacterials, anti-fungals and the like agents. Active ingredients suited for topical applications are particularly preferred.

Since in many cases the product is used as a cleansing article it is useful to add an aqueous phase which can be used as cleanser. Soils that are most difficult to clean are either water insoluble and/or strongly adhere to the skin. Therefore the liquid used as the aqueous phase is formulated such that it is capable of taking up water-insoluble materials.

### Further Phases

In another embodiment of the invention a further layer is applied to the applicator, which is made of polymeric material, hereafter referred to as polymeric layer. One or more polymeric layers may be applied to the applicator. Whenever used herein the term 'polymeric layer' refers to one or more polymeric layers.

The polymeric layer may be applied to one side of the applicator or to several sides.

The polymeric layer may be made of a suitable polymer such as polyethylene, polypropylene, polyester, a silicone and the like, including mixtures thereof. The polymeric layer may contain other materials, such as fillers or dyes. In the latter instance the area of the applicator covered with the polymeric layer will occur as colored areas. In case several polymeric layers are applied, layers with different colors may be used thus resulting in different colored patterns.

The polymeric layer may be applied to the applicator similarly as described for the application of the wax phase. For example, it may be applied continuously, i.e. over the whole surface of the applicator, or discontinuously, e.g. in patterns, e.g. as stripes, spots or other figures. In the instance where the polymeric layer does not cover the whole surface, the wax phase may cover several areas of the applicator that are covered by the polymeric layer and the other areas.

The lipid layer may be applied onto the polymeric layer thus forming a double layer. The polymeric layer needs not be completely covered by the wax phase, i.e. some parts may remain uncovered.

The polymeric layer may also be applied to the areas that are not covered by the wax phase. For example the wax phase may be applied as a layer in a discontinuous fashion and the polymeric phase is applied at the spots without wax phase. In one particular embodiment the wax phase is applied as stripes and the polymeric layer is put in the area between these stripes thus forming a pattern of alternating stripes of wax phase and polymeric layer.

The polymeric layer may be semi-solid so that it can be disrupted upon application of a product having such a layer. Semi-solid polymeric layers are made of polymers that have a waxy, creamy or similar consistency. In that instance the polymeric layer can also be applied as an external coating onto the applicator, covering one or several sides, covering parts or the whole surface. It may also cover parts or the whole of the lipid layer.

The wax phase that covers the polymeric layer may be colored or uncolored. In the former instance, the polymeric layer preferably is uncolored or white although it may be colored also. In the instance where the wax phase is uncolored, the polymeric phase preferably is colored, although it may also be white or uncolored.

The polymeric phase may be applied for improving or promoting the transfer of the wax phase that is coated thereon to the user's skin. Using a colored polymeric layer, or a colored wax phase, or both, results in an appearance, disappearance or respectively change of color when the applicator product is used and the wax phase is transferred to the skin.

The polymeric layer is applied to the applicator using art-known methods to coat materials used for manufacturing applicators like materials with a polymeric layer. For example the polymeric layer can be applied by screen printing, gravure printing, roller printing, embossing, spraying, drippling, bathing and the like techniques.

### Additional ingredients

The wax phase may contain further ingredients.

### Active ingredients

The wax phase further may contain active ingredients for application to the skin. The wax phase preferably contains oil-soluble or hydrophobic active agents. However by using suitable emulsifiers water-soluble or hydrophilic agents can be incorporated in the wax phase.

Products having a wax phase that contains one or more active ingredients constitute particularly attractive embodiments of the present invention.

The active ingredients can also be present in particular combinations.

The active ingredients can be mixed with or incorporated into suitable carriers. These comprise any skin-acceptable inert materials that are known for formulating active ingredients. The carriers can be finely or more coarsely divided powders, or even granulates. They can comprise starches, sugars, binders, lubricants, diluents, fillers, disintegrating agents, granulating agents and the like components. The nature of the carrier materials will depend on the active ingredient that is formulated therein and on the type of formulation that is desired.

Particular carriers for incorporating active ingredients are beads wherein the active ingredient is entrapped in some form. The terms 'beads' or 'polymeric beads' are meant to comprise any form of discrete, free-flowing powders, beads or capsules which envelope, coat or contain an active ingredient in a mono- or polymeric matrix or capsule. These terms are also meant to include porous beads or 'microsponges' and 'microcapsules', the latter being beads of smaller size The beads may be coated with a suitable coating material that protects the interior of the bead or controls the release of the active ingredient entrapped therein. The coating on the bead itself may contain the active ingredient in which case the coating is layed on an inert core.

Formulating an active ingredient in beads can be for protecting the active from environmental factors but is mostly done for allowing controlled release of the active.

A particular type of beads are small beads or capsules, having an average diameter which is in the micrometer range, although the average diameter can be as small as even 200 nm.

This type of capsules can be liposome-based, made for example of phospholipids such as lecithin, phosphatidyl ethanolamine, phosphatidyl serine, phosphatidic acid and the like. This type of capsules also can be made of starch, cellulose, porous gelatin and the like.

The capsules or beads can also be relatively larger, having average size in the mm or 0.1 mm range. This type of capsules or beads can be made of materials such as agar, glycolic acid polymers, and further components such as water, mineral oils, glycerin. They may contain further ingredients such as preservatives, dye(s), and the like.

Another type of beads or microcapsules are microsponges. These are materials sized from about 5 to about 300 µm (average diameter) having a large inner surface. These are obtained by polymerization of particular monomers. An active ingredient can be entrapped therein either during this polymerization process or afterwards. Microsponge-based carriers may be used to protect the active ingredient entrapped therein or for controlled release purposes.

The capsules may optionally contain one or more suitable disintegrating agents, in particular those mentioned in this specification. Upon contact with the appropriate external factor, the disintegrating agents will cause the capsules to break open thus allowing release of the active ingredient entrapped therein.

The capsules can be incorporated into the wax phase. They can also be applied to the applicator prior to the introduction of the wax phase. They can even be introduced during the manufacturing process of the applicator itself.

Release of the active from the beads or capsules can be the result of the rupture of the coating or the matrix. This may be the result of physical factors such as pressure, strain or by shearing forces upon use of the applicator product, e.g. by rubbing the product to the skin or to a surface. Release of the active ingredient may be due to the semi-permeable or porous nature of the bead or its coating or due to external factors such as contact with liquid media that cause the active ingredient to become extracted, or to dissolve or disintegrate the bead or its coating, or by temperature effects. The capsules can also be disintegrated under influence of certain chemicals, in particular by disintegrating agents incorporated into the capsules. Particular embodiments of the latter are capsules containing suitable amounts of bicarbonate and an organic acid which, upon contact with water, e.g. upon contact an aqueous phase when using the applicator product, cause the capsules to disintegrate.

The beads or capsules can be made according to methodologies generally known in the art, for example by emulsion polymerisation.

The beads or capsules may be applied to any portion of the applicator but preferably they are concentrated at the surface or in the upper surface portion of the applicator. This allows maximal transfer of the active ingredient to the skin or to the surface to which the product is applied.

The beads or capsules can be applied to the applicator in dry form by dusting, sifting, spraying and the like methods. They can also be printed or roll-coated in the form of a suitable liquid or paste. They can also be mixed with a suitable liquid, which can be a solvent that is inert towards the beads, or water, or an aqueous phase, and sprayed onto the applicator.

Examples of active agents for use in the wax phase comprise anti-microbials, e.g. anti-bacterials and antifungals, anti-inflammatory agents, anti-irritating compounds, anti-itching agents, moisturising agents, skin caring ingredients, plant extracts, vitamins, anti-inflammatories actives for anti-stinging, anti-irritants, anti-dandruffs, anti-aging or anti-wrinkling agents, skin lifting agents such as dimethyl amino ethanol (DMAE), and in particular its salt forms. Other suitable actives are e.g. Medicago officinalis, Actinidia chinensis, allantoin, Aloe barbadensis, Anona cherimolia, Anthemis nobilis, Arachis hypogaea, Arnica montana, Avena sativa, beta-carotene, bisabolol, Borago officinalis, butylene glycol, Calendula officinalis, Camellia sinensis, camphor, Candida bombicola, capryloyl glycine, Carica papaya, Centaurea cyanus, cetylpyridinium chloride, Chamomilla recutita, Chenopodium quinoa, Chinchona succirubra, Chondrus crispus, Citrus aurantium dulcis, Citrus grandis, Citrus limonum, Cocos nucifera, Coffea arabica, copper peptides such as copper tripeptide-1, Crataegus monogina, Cucumis melo, dichlorophenyl imidazoldioxolan, Enteromorpha compressa, Equisetum arvense, ethoxydiglycol, ethyl panthenol, farnesol, ferulic acid, Fragaria chiloensis, Gentiana lutea, Ginkgo biloba, glyceryl laurate, Glycyrrhiza glabra, Glycine soya, Hamamelis virginiana, heliotropine, hydrogenated palm glycerides, citrates, hydrolyzed castor oil, hydrolyzed wheat protein, Hypericum perforatum, Iris florentina, Juniperus communis, lactis proteinum, lactose, Lawsonia inermis, linalool, Linum usitatissimum, lysine, Magnesium aspartate, magnifera indica, Malva sylvestris, mannitol, mel, Melaleuca altemifolia, Mentha piperita, menthol, menthyl lactate, Mimosa tenuiflora, Nymphaea alba, olaflur, Oryza sativa, panthenol, paraffinum liquidum, PEG-20M, PEG-26 jojoba acid, PEG-26 jojoba alcohol, PEG-35 castor oil, PEG-40 hydrogenated castor oil, PEG-60 hydrogenated castor oil, PEG-8 caprylic/capric acid, Persea gratissima, petrolatum, potassium aspartate, potassium sorbate, propylene glycol, Prunus amygdalus dulcis, prunus armeniaca, Prunus persica, retinyl palmitate, Ricinus communis, Rosa canina, Rosmarinus officinalis, Rubus idaeus, salicylic acid, Sambucus nigra, sarcosine, Serenoa serrulata, Simmondsia chinensis, sodium carboxymethyl betaglucan, sodium cocoyl amino acids, sodium hyaluronate, sodium palmitoyl proline, stearoxytrimethylsilane, stearyl alcohol, sulfurized TEA-ricinoleate, talcum, thymus vulgaris, Tilia cordata, tocopherol, tocopheryl acetate, trideceth-9, Triticum vulgar, tyrosine, undecylenoyl glycine, urea, Vaccinium myrtillus, valine, zinc oxide, zinc sulfate and the like.

Of particular interest are active ingredients, that can be used for treating skin that shows inflammatory reactions, that is irritated, red or damaged. Examples of such agents are zinc compounds or sulphur.

Further active ingredients that can be used are known under the tradename Generol^{™}.These comprise ethoxylated and non-ethoxylated phytosterines. Other active ingredients comprise anti-microbial agents and biogenic active ingredients.

The active ingredients can be present, depending on the nature of the ingredients and their application, in various concentrations, but usually are present in a quantity in the range of 0,01 - 10 % (w/w), preferably from 0,1 - 7 % (w/w) and more preferably 1 - 5 % (w/w), w/w expressed to the total weight of the wax phase.

### Further additional ingredients

The wax phase may contain further ingredients such as moisturizers, refatting agents, thickeners, powders, biogenic actives, deodorants, film formers, UV sunscreen filters, anti-oxidants, hydrotropes, preservatives, insect repellents, self tanning agents, solubilizers, perfumes, dyes, pigments, and the like.

### Moisturizers

The wax phase can further contain one or more moisturizers. These are added to improve the sensoric properties as well as to regulate skin hydratation. These agents additionally can improve the penetration of the composition in or into the applicator.

Moisturizers may be present in quantities of 1 -20 % (w/w), preferably of 5 - 5 % (w/w), and more preferably 5 -10 % (w/w) -relative to the total amount of the lipid and/or aqueous phase.

Suitable moisturizers are a.o. amino acids, pyrrolidone carbonic acid, lactic acid and its salts, lactitol, urea and urea derivatives, ureic acid, glucosamine, creatinine, hydrolysis products of collagen, chitosan or chitosan salts/-derivatives, and in particular polyols and polyol derivatives (e.g. ethylene glycol, propylene glycol, butylene glycol, pentylene glycol, hexylene glycol, erythrite, 1,2,6-hexanetriol, polyethylene glycols such as PEG-4, PEG-6, PEG-7, PEG-8, PEG-9, PEG-10, PEG-12, PEG-14, PEG-16, PEG-18, PEG-20, PEG-135, PEG 150), sugar and sugar derivatives (a.o. fructose, glucose, maltose, maltitol, mannite, inosite, sorbite, sorbityl silandiol, sucrose, trehalose, xylose, xylit, glucuronic acid and its salts), ethoxylated sorbitol (Sorbeth-6, Sorbeth-20, Sorbeth-30, Sorbeth-40), honey and hydrogenated honey, hydrogenated starch hydrolysates, as well as mixtures of hydrogenated wheat protein, hydrolyzed milk protein, lecithin, pythantriol, hyaluronic acid and salts thereof, and PEG-20-acetate copolymers. Particularly preferred moisturizers are glycerine, diglycerine and triglycerine.

The addition of a dye has the advantage that it provides of a visible indication for the user, sending the message of particular (active) ingredients having been incorporated in the wax phase. It allows furthermore to visualize the stability of the phase, in particular of the wax phase, that has been applied on the applicator can be easily visualized.

### Emulsifiers

The wax phase in the products of the invention may further contain one or more emulsifiers which can be of the W/O type. The addition of an emulsifier allows the incorporation of hydrophilic components or agents into the wax phase.

Preferred are non-ionic emulsifiers which typically have good skin compatibility. Improved sensoric properties are obtained when combining non-iononics W/O and O/W emulsifiers. The wax phase may contain the emulsifier(s) in an amount of 0 to 20 % (w/w), in particular of 0.1 to 15 % (w/w), more in particular of 0.1 to 10 % (w/w), still more in particular from 0.1 to 5%, or 0.1 to 2% (w/w), relative to the total quantity of the wax phase.

### Non-ionic emulsifiers

Particular non-ionic emulsifiers comprise:
(1) Addition products of 2 to 50 moles of ethylene oxide and/or 0 to 20 moles propylene oxide to linear fatty alcohols having 8 to 40 C-atoms, to fatty acids with 12 to 40 C-atoms and to alkylphenols with 8 to 15 C-atoms in the alkyl rest.
(2) C₁₂₋₁₈-fatty acid mono- and-diesters of addition products of 1 to 50 moles of ethylene oxide and glycerine.
(3) Glycerine mono- and -diesters and sorbitan mono- and -diesters of saturated and unsaturated fatty acids with 6 to 22 C-atoms and their ethylene oxide addition products.
(4) Alkyl mono- and -oligoglycosides with 8 to 22 C-atoms in the alkyl rest and their ethoxylated analogs.
(5) Addition products of 7 to 60 moles of ethylene oxide to castor oil and/or hardened castor oil.
(6) Polyol- and in particular polyglycerine esters, such as e.g. polyol poly-12-hydroxystearate, polyglycerine polyricinoleate, polyglycerine diisostearate or polyglycerine dimerate. Also applicable are mixtures of compounds of several of these substance classes.
(7) Addition products of 2 to 15 moles of ethylene oxide to castor oil and/or hardened castor oil.
(8) Partial esters derived from linear, branch chained, unsaturated or saturated C₆-C₂₂-fatty acids, ricinoleic acid as well as 12-hydroxystearic acid and glycerine, polyglycerine, pentaerythrite, dipentaerythrit, sugar alcohols (e.g. sorbitol), alkylglucosides (e.g. methylglucoside, butylglucoside, laurylglucoside) as well as polyglucosides (e.g. cellulose), or mixed esters such as e.g. glyceryl stearate/citrate and glyceryl stearate/lactate.
(9) Wool wax alcohols.
(10) Polysiloxane-polyalkyl-polyether-copolymers and derivatives thereof.
(11) Mixed esters from pentaerythrite, fatty acids, citric acid and fatty alcohols and/or mixed esters of fatty acids with 6 to 22 C-atoms with methylglucose and polyoles, respectively glycerine or polyglycerine.
(12) Polyalkylene glycols.

The addition products of ethylene oxide and/or of propylene oxide and fatty alcohols, fatty acids, alkylphenoles, glycerine mono- and -diesters as well as sorbitan mono- and -diesters of fatty acids or of castor oil are known and commercially available products. Usually these are mixtures of homologues of which the average degree of alkoxylation corresponds to the ratio of starting quantities of ethylene oxide and/or propylene oxide and substrate, with which the addition reaction is conducted. Depending upon the degree of alkoxylation these products are either W/O- or O/W-emulsifiers. C_{12/18}-fatty acid mono- and -diesters of addition products of ethylene oxide to glycerine are known as re-fatting agents in cosmetic applications.

Particular useful and mild emulsifiers are polyolpoly-12-hydroxystearates and mixtures thereof with other components, that are available under the tradename "Dehymuls^{®} PGPH" (W/O-emulsifier) or "Eumulgin^{®} VL 75" (1:1 w/w mixture with coco-glucosides, O/W-emulsifier) or Dehymuls^{®} SBL (W/O-Emulsifier) from Cognis Deutschland GmbH. The polyol components of these emulsifiers can be derived from materials that have at least two and in particular 3 to 12 and more in particular 3 to 8 hydroxyl groups, and 2 to 12 carbon atoms.

In case it is desirable to incorporate water-soluble active ingredients and/or small amounts of water into the wax phase it can be advantageous to add an emulsifier selected from the group of non-ionic O/W-emulsifiers (HLB-value: 8 - 18) and/or solubilizers. These can for example be the already mentioned ethylene oxide-adducts with a corresponding high degree of ethoxylation e.g. 10 - 20 ethylene oxide units in the case of O/W-emulsifiers and 20 - 40 ethylene oxide units for so-called solubilizers. Particularly attractive as O/W emulsifiers are Ceteareth-12 und PEG-20 stearate. Particularly attractive solubilizers are Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether) and Eumulgin^{®} SML 20 (INCI: Polysorbate-20).

Non-ionic emulsifiers of the group of alkyl oligoglycoside are particularly skin- compatible and therefore preferred as O/W-Emulsifiers. C₈-C₂₂-alkyl mono- and - oligoglycosides, their preparation and use have been described in the prior art. Oligoglycosides are meant to comprise oligomeric glycosides with a degree of oligomerisation of up to about 8. The degree of oligerisation can also be a statistical average used for those products comprised of a specific range of oligoglycosides. An example is the product sold under the tradename Plantacare^{®} which has a C₈-C₁₆-alkyl group glycosidically bound to an oligoglucoside rest, having an average degree of oligomerisation between 1 and 2.

Other non-ionic emulsifiers are the acyl glucamides. Preferred is the product sold under the tradename Emulgade^{®} PL 68/50 (Cognis Deutschland GmbH) which is a 1:1-mixture of alkyl polyglucosides and fatty alcohols, and a mixture of lauryl glucoside, polyglyceryl-2-dipolyhydroxystearate, glycerine and water, sold under the trade name Eumulgin^{®} VL 75.

Lipophilic W/O-emulsifiers in principle are emulsifiers with a HLB-value in the range of 1 to 8. The HLB-value of ethoxylated products is calculated by the formula: HLB = (100 - L) : 5, wherein L is the percentage (in weight %) of lipophilic groups, i.e. of fatty alkyl- or fatty acyl groups in the ethylene oxide adducts.

Particularly attractive W/O-emulsifiers are the partial esters of polyoles, in particular of mono-, di- or tri-, sesqui esters of fatty acids of polyoles, more in particular of C₃-C₆-polyoles, such as, for example, glyceryl monoesters, partial esters of pentaerythrite or carbohydrate esters, e.g. saccharose distearate, or sorbitane mono-, di-, tri- or sesqui fatty esters in particular stearates, oleates, erucates, ricinoleates, hydroxystearates, isostearates (but also: tartrates, citrates, maleates) and the like. Also attractive are addition products of 1 to 30, respectively 5 to 10 moles ethylene oxide to these sorbitane esters.

### Further Surfactants/Emulsifiers

Depending upon the use of the products of the present invention, the lipid phase may further contain zwitterionic, amphoteric, cationic and or anionic surfactants.

Zwitterionic surfactants are those tensioactive compounds, that contain at least a quaternary ammonium group and at least a -COO⁽⁻⁾- or -SO₃⁽⁻⁾- group. Particularly useful zwitterionic surfactants are the so-called betaines such as N-alkyl-N,N-dimethyl ammonium glycinate, for example coco-alkyl dimethylammonium glycinate, N-acylaminopropyl-N,N-dimethylammonium glycinate, for example coco-acyl aminopropyl dimethylammonium glycinate, and 2-alkyl-3-carboxylmethyl-3-hydroxyethylimidazoline, each having 8 to 18 C-atoms in the alkyl- or acyl group as well as coco-acyl aminoethyl hydroxyethyl carboxymethyl glycinate. A preferred zwitterionic surfactant is the fatty acid amide-derivative known by its INCI-name cocamidopropyl betaine.

Ampholytic surfactants can further be added, in particular as co-surfactants. Ampholytic surfactants comprise those tensioactive compounds, that beside a C₈-C₁₈-alkyl- or acyl group at least contain a free amino group and at least a -COOH- or -SO₃H- group and are able to form internal salts. Examples of appropriate ampholytic surfactants are N-alkyl glycines, N-alkyl propionic acids, N-alkyl amino buteric acids, N-alkyl imino-dipropionic acids, N-hydroxyethyl-N-alkyl amidopropyl glycines, N-alkyl taurine, N-alkyl sarcosine, 2-alkylaminopropionic acids and alkylamino acetic acids with in each alkyl group about 8 to 18 C-atoms.

Most preferred ampholytic surfactants N-coco-alkyl aminopropionate coco-acyl amino ethylamino propionate and C₁₂₋₁₈-acylsarcosine.

Anionic surfactants are characterized by a water solubilizing anionic group such as a carboxylate-, sulfate-, sulfonate- or phosphate- group and a lipophilic rest. Particular anionic surfactants are the alkali-, ammonium- or alkanol ammonium salts of alkyl sulfates, alkyl ethersulfates, alkyl ethercarboxylates, acyl isethionates, acyl sarkosinates, acyl taurines with linear alkyl- or acyl groups having 12 to 18 C-atoms as well as alkali- or ammonium salts of sulfosuccinates and acyl glutamates.

Quaternary ammonium derivatives can in particular be used as cationic surfactants. Preferred are ammonium halogenides, in particular chlorides and bromides, e.g. alkyl trimethylammonium chloride, dialkyl dimethylammonium chloride and trialkyl methylammonium chloride, z. B. cetyl trimethylammonium chloride, stearyl trimethylammonium chloride, distearyl dimethylammonium chloride, lauryl dimethylammonium chloride, lauryl dimethylbenzylammonium chloride and tricetyl methylammonium chloride. Additional cationic surfactants are the quaternary esters with good biological degradability, such as, for example, dialkylammonium methosulfates and methylhydroxyalkyl dialkoyloxy alkylammonium methosulfates (sold under the tradename Stepantex^{®} and the products of the Dehyquart^{®}-series). The term "Esterquats" is meant to comprise quaternized fatty acid triethanolamine ester salts which have a beneficial impact on the softness of the phases, in particular of the wax phase. Further cationic surfactants are the quaternized protein hydrolysates.

### Manufacture

This invention further concerns a process for preparing a product as defined herein, said process comprising contacting the applicator with a wax phase composition as described herein and optionally drying the product. The process comprises contacting the applicator with the wax phase.

Contacting the applicator with the wax phase is as described above in the section 'wax phase', preferably by spraying, printing or by a direct contact procedure in which there is a direct contact between the applicator and an application head having slit nozzles.

A drying step may be applied at any time during the process. Drying can be done by conventional methods, e.g. by the application of hot air, or by leading the applicator through an oven or over a heated or warmed transport roll.

In case a wax phase has been applied prior to drying, the temperature of the air should be such that the wax phase does not melt. Application of air of ambient temperature may be recommendable in that instance.

The wax phase can also be applied to the applicator at any time during the manufacturing process of the applicator, for example it may be applied during the manufacturing process of the applicator material. Preferably the wax phase is applied to the applicator after finishing the manufacturing process of the applicator.

The thus obtained applicators can be packed individually or can be packed in a determined number, e.g. a number between 10 and 30 in a suitable package, for example a plastic wrap, box and the like.

Applicators with different coating and/or impregnation can be combined in one packaging. For example there can be a series of applicators with increasing or decreasing amounts of wax phase. Or colored or uncolored applicators can be alternated.

### Application and properties

The products according to the present invention advantageously result in an optimal release of the active ingredient(s) onto the skin during use.

Optimal release of active ingredients can be achieved by using a wax phase which is a solid lipid having a melting point or melting range which is equal to or slightly exceeds body temperature. Without being bound to theory, it is believed that this results in a quicker melting of the wax phase causing a faster and more efficient transfer and release to the skin of the active materials.

Optimal release of active ingredients can also be achieved by using a suitable emulsifier in the lipid phase to cause a local emulsification process on the skin during use of the applicators. This local emulsification can also be achieved by contacting the wax phase in the products with water or with an aqueous phase prior to usage. Or this local emulsification is achieved by using the products on a wet skin. This local emulsification may be the result of body temperature causing the wax phase to melt or it may be the result of pressure exerted during usage of the wipe, or it may be the result of both, the latter being usually the case. In the instance of local emulsification by the effect of pressure, the emulsification process is driven by the (limited) pressure exerted by the user when applying the wipe, e.g. by rubbing it across the skin, dabbing it and the like. This causes the lipid phase to come in contact with water or an aqueous phase and form an emulsion locally.

In this local emulsification process, a limited amount of the phase without emulsifier is incorporated into the phase having the emulsifier.
Although in preferred executions the wax phase is not present on the whole surface of the wipe, good release of the wax phase and of the components contained therein is attained, in particular when the local emulsification process comes into play.

Optimal release of active ingredients can also be achieved by making use of both above possibilities.

The products of the invention can be for use as end products. In this instance the consumer is instructed to treat these products with water or with an aqueous lotion which for example may be sold separately.

Or they can be used as such, e.g. as a dry applicator for use on a wet skin.

The products of the invention may also find use as intermediate products for making applicators having a wax phase. They can be stored or transported to other sites for further handling.

The products according to the invention can be for baby or adult use in a wide range of applications as personal care products, comprising, for example, baby cleansing, face or body cleansing, skin treatment or skin conditioning such as for example skin moisturization and against skin aging, insect repellence, powder applicators, toilet applicators, anti-perspirant applicators, peeling applicators, after-sun treatment, sunscreen applicators, applicators for feminine hygiene, nappy rash applicators, the latter preferably containing zinc oxide as active ingredient, and the like.

The products of the present invention have a low water content, for example a water content which is below 10 %, or lower w/w relative to the total weight of the product. Examples of products with low water content are the so-called dry applicators which are aimed for use on a wet skin. Examples of applications for this type of applicators are usage in the shower or after bathing. Such dry applicators may also be recommended for use after wetting the product itself, e.g. with water or with an aqueous lotion that is provided separately.

The products of the invention may find use as cleansing tools, in particular when wetted, however their use is not limited to this application only. They are particularly effective when wetted with water, which is due, i.a., by the fact that they can remove both aqueous and lipid soils and components. The products of the invention may in particular be used as cleansers for babies because of their effectiveness to remove waste deposits, as well as to reduce a number of micro-organisms that can cause infection. The products described herein find use as applicators of active substances, in particular of the active substances mentioned herein, or they find use as both cleanser and applicator of active substances in one product.

The products of this invention have excellent transfer of active ingredients to the skin thus widening the applications of applicator products as a vehicle for a number of actives, in particular more expensive actives that so far could not be applied because of poor transfer rate. The products of this invention not only provide a more efficient transfer of active ingredients to the skin, but moreover provide other consumer benefits such as a more even distribution of the actives on the skin, better skin penetration.

The products of this invention show the additional advantage that they may combine in one and the same product both cleansing capability and the transfer of active ingredients to the skin, i.e. the application of leave-on products. They further allow to independently optimize the cleansing and skincare attributes of the product and at the same time improve the delivery of skincare actives onto the skin. Hence, either of both aspects may be present in a larger extend, i.e. the product may be primarily for cleansing purposes but also having the capability of transferring beneficial components or active substances to the skin, or vice versa, the products may be designed for applications in instances where the primary benefit is not cleansing but a better and more convenient form of application of leave-on products.

The products of the invention therefore show improved performance in terms of cleansing and skin benefits since both attributes can be formulated in different phases independently.

Another benefit of the products of this invention is that they may offer a softer feel of the applicator material due to the modification of the applicator surface caused by the presence of the wax phase. The products moreover offer gentler cleansing because of less friction of the applicator on the skin (softer skin-feel).

A still further advantage lies in the fact that the instant products allow an improved transfer of actives onto the skin since the active ingredients usually are concentrated at the surface of the applicator material and not included in the inner phase of a typical o/w-emulsion.

Most types of wax phases described in this specification, possess the additional advantage that they are almost odorless (unless fragrances are added), environmentally friendly and biologically decomposable.

The products of this invention are particularly attractive because they allow convenient and quick application and an easy and more evenly distribution of any ingredient incorporated therein or thereon. They moreover are for application on babies and children. The products additionally allow faster and effective cleansing.

In view of these beneficial properties, the products of this invention can be used in a wide variety of cosmetic and personal care applications, but also in other cleaning or cleansing applications such as cleaning of hard surfaces.

### Examples

The following examples are given with the nomenclature of INCI. As used in the following examples, C.I. refers to dyes.

### Example 1: wax phases

### Phase 1-A

| | |
|---|---|
| Cocoglycerides | 64.99 % |
| Cetyl Alcohol | 33.00 % |
| Di-Stearyl Ether | 1.00 % |
| Tocopherol | 1.00 % |
| C.I. 61565 | 0.01% |

### Phase 1-B

| | |
|---|---|
| Cocoglycerides | 54.99 % |
| Cetyl Alcohol | 33.00 % |
| Ceteareth-12 | 3.00 % |
| Glyceryl Stearate | 4.00 % |
| Di-Stearyl Carbonate | 2.00 % |
| Tocopherol | 1.00 % |
| C.I. 61565 | 0.01 % |
| Aqua | 2.00 % |

### Phase 1-C

| | |
|---|---|
| Cocoglycerides | 49.99 % |
| Cetearyl Alcohol | 20.00 % |
| Cegesoft® HF 52 | 5.00 % |
| Cegesoft® PS 6 | 3.00 % |
| Ceteareth-12 | 2.00 % |
| Glyceryl Stearate | 2.00 % |
| PEG-20 Stearate | 10.00 % |
| Di-Stearyl Ether | 2.00 % |
| Tocopherol | 1.00 % |
| C.I. 61565 | 0.01 % |
| Aqua | 5.00 % |

### Phase 1-D

| | |
|---|---|
| Cocoglycerides | 58.99 % |
| Glyceryl Stearate | 25.00 % |
| Glyceryl Laurate | 14.00 % |
| Di-Stearyl Carbonate | 1.00 % |
| Tocopherol | 1.00 % |
| C.I. 75300 | 0.01 % |

### Phase 1-E

| | |
|---|---|
| Cocoglycerides | 30.00 % |
| Cetearyl Alcohol | 1.00 % |
| Cegesoft® HF 52 | 20.00 % |
| Cegesoft® GPO | 5.00 % |
| Ceteareth-12 | 15.00 % |
| Glyceryl Stearate | 20.00 % |
| Di-Stearyl Ether | 5.00 % |
| Tocopherol | 1.00 % |
| Panthenol | 1.00 % |
| Aqua | 2.00 % |

### Phase 1-F

| | |
|---|---|
| Coco glycerides | 19.99 % |
| Cetearyl Alcohol | 30.00 % |
| Cegesoft® PS 6 | 10.00 % |
| Eumulgin® VL 75 | 10.00 % |
| Ceteareth-12 | 5.00 % |
| Glyceryl Stearate | 10.00 % |
| Di-Stearyl Carbonate | 5.00 % |
| Tospearl® 145 A | 5.00 % |
| Zinc Stearate | 2.00 % |
| C.I. 61565 | 0.01 % |
| Aqua | 3.00 % |

### Phase 1-G

| | |
|---|---|
| Myristyl Alcohol | 19.99 % |
| Cocoglycerides | 10.00 % |
| Cegesoft® HF 52 | 20.00 % |
| Eumulgin® VL 75 | 10.00 % |
| Glyceryl Stearate | 20.00 % |
| PEG-20 Stearate | 5.00 % |
| Di-Stearyl Carbonate | 2.00 % |
| Panthenol | 3.00 % |
| C.I. 61565 | 0.01 % |
| Aqua | 10.00 % |

### Phase 1-H

| | |
|---|---|
| Myristyl Alcohol | 47.99 % |
| Stearyl Alcohol | 25.00 % |
| Eumulgin® VL 75 | 2.00 % |
| PEG-20 Stearate | 14.00 % |
| 1,2-Hexadecanediol | 5.00 % |
| Bisabolol | 1.00 % |
| C.I. 47000 | 0.01 % |
| Aqua | 5.00 % |

### Phase 1-I

| | |
|---|---|
| Cocoglycerides | 47.99 % |
| Stearyl Alcohol | 20.00 % |
| Eumulgin® VL 75 | 2.00 % |
| PEG-20 Stearate | 12.00 % |
| Di-Stearyl Carbonate | 5.00 % |
| Cyclomethicone | 3.00 % |
| Tospearl® 145 A | 5.00 % |
| C.L 75300 | 0.01 % |
| Aqua | 5.00% |

### Phase 1-J

| | |
|---|---|
| Cocoglycerides | 55.99 % |
| Glyceryl Stearate | 20.00 % |
| Glyceryl Laurate | 15.00 % |
| Di-Stearyl Carbonate | 5.00 % |
| Talc | 2.00 % |
| Aluminum Starch Octenylsuccinate | 2.00 % |
| C.I. 60725 | 0.01 % |

### Phase 1-K

| | |
|---|---|
| Cocoglycerides | 50.99 % |
| Glyceryl Stearate | 25.00 % |
| Glyceryl Laurate | 15.00 % |
| Di-Stearyl Ether | 5.00 % |
| Talc | 2.00 % |
| Timiron® Splendid Gold | 2.00 % |
| C.I. 21230 | 0.01 % |

### Phase 1-L

| | |
|---|---|
| Myristyl Alcohol | 58.99 % |
| Stearyl Alcohol | 23.00 % |
| PEG-20 Stearate | 15.00 % |
| Di-Stearyl Carbonate | 2.00 % |
| Panthenol | 1.00 % |
| C.I. 61525 | 0.01 % |

### Phase 1-M

| | |
|---|---|
| Myristyl Alcohol | 47.99 % |
| Stearyl Alcohol | 25.00 % |
| Eumulgin® VL 75 | 2.00 % |
| PEG-20 Stearate | 10.00 % |
| Di-Stearyl Ether | 7.00 % |
| Panthenol | 2.00 % |
| C.I. 61525 | 0.01 % |
| Aqua | 6.00 % |

### Phase 1-N

| | |
|---|---|
| Myristyl Alcohol | 50.00 % |
| Stearyl Alcohol | 25.00 % |
| Eumulgin® VL 75 | 2.00 % |
| PEG-20 Stearate | 10.00 % |
| Di-Stearyl Ether | 7.00 % |
| Ethyl Butylacetylaminopropionate | 5.00 % |
| Panthenol | 1.00 % |

### Phase 1-O

| | |
|---|---|
| Cocoglycerides | 54.99 % |
| Cetyl Alcohol | 33.00 % |
| Ceteareth-12 | 3.00 % |
| Glyceryl Stearate | 4.00 % |
| Di-Stearyl Carbonate | 2.00 % |
| Octyl Methoxycinnamate | 6.00 % |
| C.I. 61565 | 0.01 % |

### Phase 1-P

| | |
|---|---|
| Cocoglycerides | 56.99 % |
| Glyceryl Stearate | 25.00 % |
| Glyceryl Laurate | 14.00 % |
| Di-Stearyl Carbonate | 1.00 % |
| Polyethylene | 3.00 % |
| C.I. 75300 | 0.01 % |

### Phase 1-Q

| | |
|---|---|
| Cocoglycerides | 58.93 % |
| Glyceryl Stearate | 25.00 % |
| Glyceryl Laurate | 15.00 % |
| Di-Stearyl Ether | 1.00 % |
| Aqua | 0.06 % |
| C.I. 61565 | 0.01 % |

### Phase 1-R

| | |
|---|---|
| Cocoglycerides | 43.93 % |
| Stearyl Alcohol | 15.00 % |
| Glyceryl Stearate | 25.00 % |
| Glyceryl Laurate | 15.00 % |
| Di-Stearyl Ether | 1.00 % |
| Aqua | 0.06 % |
| C.L 61565 | 0.01 % |

### Phase 1-S

| | |
|---|---|
| Cocoglycerides | 44.93 % |
| Glyceryl Stearate | 25.00 % |
| Glyceryl Laurate | 15.00 % |
| Di-Stearyl Ether | 15.00 % |
| Aqua | 0.06 % |
| C.I. 61565 | 0.01 % |

### Example 2:

Dry sponge consisting of two parts made of different material are glued together. One part is made of liquid cellulose. After drying, the sponge material forms a layer with the thickness of 37 mm. The sponge has a surface weight of 70 g/m2 and the material is cut into blocks of 135 x 90 x 37 mm. The other part of the product is made of polyurethane with the measures 135 x 90 x 5 mm. After gluing both parts together a wax phase as described in set four of example list 1 was applied with 5 g/article onto the polyurethane side. The product is wrapped into single packs.

### Example 3:

Dry sponge made of a mixture of liquid cellulose and a wax phase, which is prepared as set two in example 1. The sponge has a surface weight of 70 g/m2 and was cut after forming into blocks of 135 x 90 x 37 mm. Lipid addition to the cellulose was set at 5%. The product is wrapped into a single pack.

## Claims

1. A product comprising an applicator, other than a porous or absorbent sheet, whereto a wax phase has been applied, wherein the wax phase has a melting point or melting range equal or above 25°C and is a composition comprising at least a wax component selected from waxy, saturated C₁₆₋₃₀-dialkyl ethers, C₁₄₋₃₀-dialkyl(ene) carbonates, and C₉₋₃₄-dicarboxylic acids or mixtures thereof.

2. A product according to claim 1 wherein the melting point or melting range of the wax phase is in the range of 32 to 40°C.

3. A product according to claim 1 or 2 wherein the wax phase additionally comprises mono-, di-or triglycerides.

4. A product according to claim 3 wherein the wax phase additionally comprises mono-, di-or triglycerides derived from or present in natural oils.

5. A product according to claim 3 wherein the wax phase additionally comprises fatty acid mono-, di-or triglycerides wherein the fatty acids contain from 12 to 24, preferably from 16 to 20 carbon atoms.

6. A product according to claim 3 wherein the wax phase comprises triglycerides selected from glyceryl stearate, glyceryl oleate, glyceryl laurate, glyceryl myristate, cocoglycerides, or hydrogenated palm oil glycerides, hydrogenated castor oil, or hydrogenated rapeseed oil.

7. A product according to claim 1 or 2 wherein the wax phase additionally contains fatty alcohols.

8. A product according to claim 7 wherein the wax phase contains C₁₂-C₅₀-fatty alcohols, in particular the C₁₂-C₂₄-fatty alcohols.

9. A product according to claim 8 wherein the fatty alcohols are selected from myristyl alcohol, 1-pentadecanol, cetyl alcohol, lauryl alcohol, oleyl alcohol, palmityl alcohol, 1-heptadecanol, stearyl alcohol, cetearyl alcohol, 1-nonadecanol, arachidyl alcohol, 1-heneicosanol, behenyl alcohol, brassidyl alcohol, lignoceryl alcohol, ceryl alcohol or myricyl alcohol and C₆-C₈-Guerbet alcohols.

10. A product according to claim 8 or 9 wherein the fatty alcohols are present in the wax phase, in an amount relative to the total weight amount of the wax phase, which is in the range of 1-40 %, preferably 1-30 % (w/w), more preferably of 1-20 %(w/w), still more preferably from 1-10 % (w/w).

11. A product according to claim 1 or 2 wherein the wax phase contains fatty acids.

12. A product according to claim 11 wherein the fatty acids are C₁₄-C₄₀-fatty acids or in particular are C₁₆-C₃₀-fatty acids.

13. A product according to any of claims 10 to 12 wherein the total amount of the fatty acids present in the wax phase, relative to the total weight amount of the wax phase, is in the range of 1-30 % (w/w), preferably of 1-20 % (w/w), more preferably from 1- 10 %(w/w).

14. A product according to claim 1 or 2 wherein the wax phase additionally contains one or more of components (a), (b), (c),(d), (e) or(f) as defined hereafter: (a) at least 1-50% (w/w), in particular at least 1-10 % of an oily or waxy component, (b) 0.1-5 % (w/w) of at least one active ingredient, (c) 1-10 % (w/w) of at least one oil, (d) 0.1-10 % (w/w) of at least one emulsifier, (e) 5-90 % (w/w) of further waxy components, and (f) 0-5 % (w/w) water.

15. A product according to claim 14 wherein the wax phase contains all components (a)- (f).

16. A product according to any of claims 1 to 15 wherein the wax phase contains one or more active substances.

17. A product according to claim 16 wherein the active substance (s) is or are anti-microbials, e. g, anti-bacterials and antifungals, anti-inflammatory agents, anti-irritating, anti-itching, anti-perspirant agents.

18. A product according to any of claims 1 to 15 wherein the wax phase contains at least one moisturizer, deodorant, skin caring ingredient, plant extract, vitamin, perfume oil, dye, sunscreen filter, hydrotrope or self-tanning agent.

19. A product according to any of claims 1 to 15, wherein the wax phase contains at least one emulsifier, superfatting agent, thickener, cationic polymer, aniomic polymer, zwitterionic polymer, amphoteric polymer, consistency agent, anti-oxidant, an insect repellent, a sunscreen filter, a powder or a peeling agent.

20. A product according to any of claims 1 to 19 which is a puff (pouf), pad, sponge, cotton ball, swab, brush, glove, mitt or bar.

21. A product according to claim 20 wherein puff or pad, a sponge or a bar is wrapped in a layered material.

22. A method of manufacturing a product as claimed in any of claims 1 to 21 said method comprising contacting the applicator with a wax phase.

23. A method according to claim 22 wherein the wax phase is applied by spraying, contacting, printing or a direct contact process where there is a direct contact between the applicator and an application head having slit nozzles.

24. Use of a product as claimed in any of claims 1 to 21 as a combined cleanser and applicator of active substances.

## Patentansprüche

1. Produkt, das einen Applikator umfasst, bei dem es sich nicht um ein poröses oder absorptionsfähiges Flächengebilde ("sheet") handelt, worauf eine Wachsphase aufgebracht wurde, wobei die Wachsphase einen Schmelzpunkt oder Schmelzbereich gleich oder über 25°C aufweist und eine Zusammensetzung ist, die mindestens eine Wachskomponente umfasst, ausgewählt aus wachsartigen, gesättigten C₁₆₋₃₀-Dialkylethern, C₁₄₋₃₀-Dialkyl/alkencarbonaten und C₉₋₃₄-Dicarbonsäuren oder Gemischen derselben.

2. Produkt nach Anspruch 1, **dadurch gekennzeichnet, dass** der Schmelzpunkt oder Schmelzbereich der Wachsphase im Bereich von 32 bis 40°C liegt.

3. Produkt nach Anspruch 1 oder 2, **dadurch gekennzeichnet, dass** die Wachsphase zusätzlich Mono-, Di- oder Triglyceride umfasst.

4. Produkt nach Anspruch 3, **dadurch gekennzeichnet, dass** die Wachsphase zusätzlich Mono-, Di- oder Triglyceride umfasst, die von natürlichen Ölen abgeleitet sind oder in diesen vorliegen.

5. Produkt nach Anspruch 3, **dadurch gekennzeichnet, dass** die Wachsphase zusätzlich Fettsäuremono-, -di- oder -triglyceride umfasst, wobei die Fettsäuren 12 bis 24, vorzugsweise 16 bis 20 Kohlenstoffatome enthalten.

6. Produkt nach Anspruch 3, **dadurch gekennzeichnet, dass** die Wachsphase Triglyceride umfasst, ausgewählt aus Glycerylstearat, Glyceryloleat, Glyceryllaurat, Glycerylmyristat, Cocoglyceriden oder hydrierten Palmölglyceriden, hydriertem Rizinusöl oder hydriertem Rapsöl.

7. Produkt nach Anspruch 1 oder 2, **dadurch gekennzeichnet, dass** die Wachsphase zusätzlich Fettalkohole enthält.

8. Produkt nach Anspruch 7, **dadurch gekennzeichnet, dass** die Wachsphase C₁₂₋₅₀-Fettalkohole, insbesondere die C₁₂-C₂₄-Fettalkohole, enthält.

9. Produkt nach Anspruch 8, **dadurch gekennzeichnet, dass** die Fettalkohole ausgewählt sind aus Myristylalkohol, 1-Pentadecanol, Cetylalkohol, Laurylalkohol, Oleylalkohol, Palmitylalkohol, 1-eptadecanol, Stearylalkohol, Cetearylalkohol, 1-änadecanol, Arachidylalkohol, 1-Heneicosanol, Behenylalkohol, Brassidylalkohol, Lignocerylalkohol, Cerylalkohol oder Myricylalkohol und C₆-C₈-Guerbetalkoholen.

10. Produkt nach Anspruch 8 oder 9, **dadurch gekennzeichnet, dass** die Fettalkohole in der Wachsphase vorliegen, in einer Menge bezogen auf die Gesamtgewichtsmenge der Wachsphase, die im Bereich von 1-40 %, vorzugsweise 1-30 % (Gew./Gew.), bevorzugter 1-20 % (Gew./Gew.), noch bevorzugter 1-10 % (Gew./Gew.), liegt.

11. Produkt nach Anspruch 1 oder 2, **dadurch gekennzeichnet, dass** die Wachsphase Fettsäuren enthält.

12. Produkt nach Anspruch 11, **dadurch gekennzeichnet, dass** die Fettsäuren C₁₄-C₄₀-Fettsäuren oder insbesondere C₁₆-C₃₀-Fettsäuren sind.

13. Produkt nach einem der Ansprüche 10 bis 12, **dadurch gekennzeichnet, dass** die Gesamtmenge der in der Wachsphase vorliegenden Fettsäuren bezogen auf die Gesamtgewichtsmenge der Wachsphase im Bereich von 1-30 % (Gew./Gew.), vorzugsweise 1-20 % (Gew./Gew.), bevorzugter 1-10 % (Gew./Gew.), liegt.

14. Produkt nach Anspruch 1 oder 2, **dadurch gekennzeichnet, dass** die Wachsphase zusätzlich eine oder mehrere Komponenten (a), (b), (c), (d), (e) oder (f), wie nachstehend definiert, enthält: (a) mindestens 1-50 % (Gew./Gew.), insbesondere mindestens 1-10 %, einer öl- oder wachsartigen Komponente, (b) 0,1-5 % (Gew./Gew.) mindestens eines wirksamen Inhaltsstoffs, (c) 1-10 % (Gew./Gew.) mindestens eines Öls, (d) 0,1-10 % (Gew./Gew.) mindestens eines Emulgators, (e) 5-90 % (Gew./Gew.) weiterer wachsartiger Komponenten und (f) 0-5 % (Gew./Gew.) Wasser.

15. Produkt nach Anspruch 14, **dadurch gekennzeichnet, dass** die Wachsphase alle Komponenten (a)-(f) enthält.

16. Produkt nach einem der Ansprüche 1 bis 15, **dadurch gekennzeichnet, dass** die Wachsphase einen oder mehrere Wirkstoffe enthält.

17. Produkt nach Anspruch 16, **dadurch gekennzeichnet, dass** der/die Wirkstoff(e) antimikrobielle Stoffe, z. B. antibakterielle Stoffe und Antimykotika, entzündungshemmende Stoffe, reizlindernde, juckreizlindernde, schweißhemmende Mittel ist/sind.

18. Produkt nach einem der Ansprüche 1 bis 15, **dadurch gekennzeichnet, dass** die Wachsphase mindestens ein(en) Feuchthaltemittel, Desodorans, hautpflegenden Inhaltsstoff, Pflanzenextrakt, Vitamin, Parfümöl, Farbstoff, Sonnenschutzfilter, Hydrotropikum oder Selbstbräunungsmittel enthält.

19. Produkt nach einem der Ansprüche 1 bis 15, **dadurch gekennzeichnet, dass** die Wachsphase mindestens ein(en) Emulgator, überfettendes Mittel, Verdickungsmittel, kationisches Polymer, anionisches Polymer, zwitterionisches Polymer, amphoteres Polymer, Konsistenzmittel, Antioxidans, ein insektenabwehrendes Mittel, einen Sonnenschutzfilter, einen Puder oder ein Peelingmittel enthält.

20. Produkt nach einem der Ansprüche 1 bis 19, das ein(e) Puderquaste, Pad, Schwamm, Wattebausch, Tupfer, Bürste, Fingerhandschuh, Fausthandschuh oder Barren ist.

21. Produkt nach Anspruch 20, **dadurch gekennzeichnet, dass** Puderquaste oder Pad, ein Schwamm oder ein Barren in ein Schichtmaterial eingewickelt ist.

22. Verfahren zur Herstellung eines Produkts nach einem der Ansprüche 1 bis 21, wobei das Verfahren das In-Kontakt-Bringen des Applikators mit einer Wachsphase umfasst.

23. Verfahren nach Anspruch 22, **dadurch gekennzeichnet, dass** die Wachsphase durch Sprühen, In-Kontakt-Bringen, Drucken oder einen Direktkontaktprozess, bei dem ein direkter Kontakt zwischen dem Applikator und einem Applikationskopf mit Schlitzdüsen besteht, aufgebracht wird.

24. Verwendung eines Produkts nach einem der Ansprüche 1 bis 21 als kombinierter Reiniger und Wirkstoffapplikator.

## Revendications

1. Produit comprenant un applicateur, autre qu'une feuille absorbante ou poreuse, sur lequel une phase cire a été appliquée, la phase cire ayant un point de fusion ou une plage de fusion égal(e) ou supérieur(e) à 25°C et étant une composition comprenant au moins un composant de cire sélectionné parmi des éthers de dialkyle en C₁₆₋₃₀ saturés cireux, des carbonates de dialkyl(ène) en C₁₄₋₃₀, et des acides dicarboxyliques en C₉₋₃₄ cireux ou des mélanges de ceux-ci.

2. Produit selon la revendication 1, dans lequel le point de fusion ou la plage de fusion de la phase cire est dans la plage de 32 à 40°C.

3. Produit selon la revendication 1 ou 2, dans lequel la phase cire supplémentaire comprend des monoglycérides, diglycérides ou triglycérides.

4. Produit selon la revendication 3, dans lequel la phase cire supplémentaire comprend des monoglycérides, diglycérides ou triglycérides dérivés à partir ou présents dans des huiles naturelles.

5. Produit selon la revendication 3, dans lequel la phase cire supplémentaire comprend des monoglycérides, diglycérides ou triglycérides d'acide gras dans lesquels les acides gras contiennent de 12 à 24, de préférence de 16 à 20 atomes de carbone.

6. Produit selon la revendication 3, dans lequel la phase cire comprend des triglycérides sélectionnés parmi le stéarate de glycéryle, l'oléate de glycéryle, le laurate de glycéryle, le myristate de glycéryle, des cocoglycérides, ou des glycérides d'huile de palme hydrogénée, l'huile de ricin hydrogénée, ou l'huile de colza hydrogénée.

7. Produit selon la revendication 1 ou 2, dans lequel la phase cire supplémentaire contient des alcools gras.

8. Produit selon la revendication 7, dans lequel la phase cire contient des alcools gras en C₁₂-C₅₀, en particulier les alcools gras en C₁₂-C₂₄.

9. Produit selon la revendication 8, dans lequel les alcools gras sont sélectionnés parmi l'alcool myristique, le 1-pentadécanol, l'alcool cétylique, l'alcool laurylique, l'alcool oléylique, l'alcool palmitique, le 1-heptadécanol, l'alcool stéarylique, l'alcool cétéarylique, le 1-nonadécanol, l'alcool arachidylique, le 1-hénéicosanol, l'alcool béhénylique, l'alcool brassidylique, l'alcool lignocérylique, l'alcool cérylique, ou l'alcool myricylique, et les alcools de Guerbet en C₆-C₈.

10. Produit selon la revendication 8 ou 9, dans lequel les alcools gras sont présents dans la phase cire, en une quantité par rapport au poids total de la phase cire qui est dans la plage de 1 à 40 %, de manière préférée de 1 à 30 % (poids/poids), de manière plus préférée de 1 à 20 % (poids/poids), de manière encore plus préférée de 1 à 10 % (poids/poids).

11. Produit selon la revendication 1 ou 2, dans lequel la phase cire contient des acides gras.

12. Produit selon la revendication 11, dans lequel les acides gras sont des acides gras en C₁₄-C₄₀ ou sont en particulier des acides gras en C₁₆-C₃₀.

13. Produit selon l'une quelconque des revendications 10 à 12, dans lequel la quantité totale des acides gras présents dans la phase cire, par rapport au poids totale, est dans la plage de 1 à 30 % (poids/poids), de manière préférée de 1 à 20 % (poids/poids), de manière plus préférée de 1 à 10 % (poids/poids).

14. Produit selon la revendication 1 ou 2, dans lequel la phase cire contient de manière supplémentaire un ou plusieurs composants (a), (b), (c), (d), (e), ou (f) tels que définis ci-dessus : (a) au moins 1 à 50 % (poids/poids), en particulier au moins 1 à 10 % d'un composant huileux ou cireux, (b) 0,1 à 5 % (poids/poids) d'au moins un ingrédient actif, (c) 1 à 10 % (poids/poids) d'au moins une huile, (d) 0,1 à 10 % (poids/poids) d'au moins un émulsifiant, (e) 5 à 90 % (poids/poids) de composants cireux additionnels, et (f) 0 à 5 % (poids/poids) d'eau.

15. Produit selon la revendication 14, dans lequel la phase cire contient tous les composants (a) à (f).

16. Produit selon l'une quelconque des revendications 1 à 15, dans lequel la phase cire contient une ou plusieurs substances actives.

17. Produit selon la revendication 16, dans lequel la ou les substance(s) active(s) est ou sont antimicrobienne(s), par exemple des substances antibactériennes et des substances antifongiques, des agents anti-inflammatoire(s), des agents anti-irritants, des agents antihistaminiques, des agents antitranspirants.

18. Produit selon l'une quelconque des revendications 1 à 15, dans lequel la phase cire contient au moins une crème hydratante, un déodorant, un ingrédient de soin de la peau, un extrait de plante, une vitamine, une huile parfumée, une matière colorante, un filtre solaire, un agent hydrotrope ou autobronzant.

19. Produit selon l'une quelconque des revendications 1 à 15, dans lequel la phase cire contient au moins un émulsifiant, un agent surgraissant, un épaississant, un polymère cationique, un polymère anionique, un polymère zwitterionique, un polymère amphotère, un agent de consistance, un antioxydant, un insectifuge, un filtre solaire, une poudre ou un agent de pelage.

20. Produit selon l'une quelconque des revendications 1 à 19, qui est une houppette, une compresse, une éponge, un tampon d'ouate, un coton-tige, une moufle ou une barrette.

21. Produit selon la revendication 20, dans lequel une houppette ou une compresse, une éponge ou une barrette est enveloppé(e) dans un matériau stratifié.

22. Procédé de fabrication d'un produit selon l'une quelconque des revendications 1 à 21, ledit procédé comprenant la mise en contact de l'applicateur avec une phase cire.

23. Procédé selon la revendication 22, dans lequel la phase cire est appliquée par pulvérisation, mise en contact, impression ou un processus de contact direct dans lequel il y a un contact direct entre l'applicateur et une tête d'application présentant des buses à fente.

24. Utilisation d'un produit selon l'une quelconque des revendications 1 à 21 en tant que dispositif de nettoyage combiné à un applicateur de substances actives.
